# EUROPÄISCHE PATENTSCHRIFT

(11) **EP 0 471 252 B1**
(45) Veröffentlichungstag und Bekanntmachung des Hinweises auf die Patenterteilung: **30.03.1994**
(21) Anmeldenummer: 91113021.9
(22) Anmeldetag: 02.08.1991
(51) Int. Cl.: C07C 233/69, C07D 307/89, C07D 307/92, A61K 6/00

(54) **N-Alkyl-N-(meth)acryloyloxyalkylcarboxamide aromatischer Carbonsäuren und aromatischer Carbonsäureanhydride sowie Adhäsive enthaltend diese Verbindungen**
N-alkyl-N-(meth)acryloyloxyalkylcarboxamides of aromatic carboxylic acids and of aromatic carboxylic anhydrides and adhesives containing these compounds
N-alkyl-N-(méth)acryloyloxyalkylcarboxamides d'acides carboxyliques aromatiques et d'anhydrides d'acides carboxyliques aromatiques ainsi qu'adhésifs contenant ces composés

(30) Priorität: 15.08.1990 DE 4025784; 11.12.1990 DE 4039440
(43) Veröffentlichungstag der Anmeldung: 19.02.1992
(73) Patentinhaber: BAYER AG, 51368 Leverkusen (DE)
(72) Erfinder: Müller, Michael, Dr., W-5060 Bergisch Gladbach 2 (DE); Podszun, Wolfgang, Dr., W-5000 Köln 80 (DE); Finger, Werner, Prof. Dr., W-4047 Dormagen 2 (DE); Winkel, Jens, Dr., W-5000 Köln 71 (DE)

(56) Entgegenhaltungen:
- EP-A- 0 377 072
- DE-A- 3 510 962
- US-A- 4 148 988

## Beschreibung

Die Erfindung betrifft neue N-Alkyl-N-(meth)acryloyloxyalkylcarboxamide aromatischer Carbonsäuren und aromatischer Carbonsäureanhydride, ihre Herstellung sowie Zubereitungen dieser Verbindungen zur Verwendung als Adhäsive zur Behandlung von Zahnhartsubstanz.

Ein besonders gravierendes Problem in der konservierenden Zahnheilkunde besteht in der dauerhaften, randspaltfreien Verklebung von Kunststoff-Füllungsmaterialien mit der Zahnhartsubstanz (Dentin und Zahnschmelz). Im Dentalbereich werden härtende, polymere Materialien als Füllungsmaterialien bei Zahnreparaturen verwendet. Als härtende, polymere Materialien werden im allgemeinen Füllungen auf Acrylatbasis bevorzugt, die während der Aushärtung schrumpfen und so zur Randspaltbildung beitragen.

Diese polymeren Füllungen haben weiterhin den Nachteil, daß sie schlecht am Dentin haften bleiben. Um dieses Problem zu lösen, hat man bisher teilweise Unterschneidungen am Zahnbein vorgenommen; dazu war es erforderlich, über den angegriffenen Bereich hinaus beachtliche Mengen an frischem Dentin zu entfernen. Nach einer anderen Methode ätzt man das Dentin und die Schmelzoberfläche mit Säuren, wie z.B. Phosphorsäure, an und nimmt dann die Füllung vor. Abgesehen davon, daß die Säure eine Reizwirkung im Mundbereich ausübt, dringt sie auch leicht durch die Dentinkanäle in den Zahn und schädigt den Nerv (Pulpa). Als Haftvermittler für Füllungen im Dentalbereich werden in der US 4, 148, 988 Substanzgemische von Trimellitsäure-4-methacryloyloxyethylester (4-MET) oder Trimellitsäureanhydrid-4-methacryloyloxyethylester (4-META) mit ethylenisch ungesättigten Monomeren und Radikalinitiatoren beschrieben. Ein sich aus dem 4-META aufbauendes Handelsprodukt (Superbond von Sun Medical) muß zum Erhalt der applikationsfertigen Form mit Methylmethacrylat (MMA), Polymethylmethacrylat (PMMA) und teilweise oxidiertem Tri-n-butylboran (TBB) abgemischt werden (MMA-4-META-TBB-Resin / Y.-S. Kuo, Proc. Natl. Sci., Counc ROC (B) Vol. 8, No. 2 (1984), 187 - 192). Ähnlich kompliziert aufgebaute Anmischungen des 4-METAs werden auch beschrieben unter Zusatz eines polyfunktionellen (Meth)acrylates (EP 266 220). Entsprechende Anmischungen mit isomeren Naphthalintricarbonsäureanhydrid-methacryloyloxyethylestern (4-MENTA) sind ebenfalls bekannt (I. Harashima et al, Dental Materials Journal 7, 2 (1988) 141 - 150). Zwar führt die DOS 3510962 aus, daß eine Chloroform-Lösung des 4-MENTAs auch ohne weitere Zusätze verwendet werden kann, jedoch ist dann ein zusätzlicher Arbeitsschritt notwendig, um MMA, PMMA und TBB nachträglich zu applizieren. Alle diese Haftvermittler sind nicht in der Lage, randspaltfreie Füllungen zu erzeugen; auch ein optimiertes, photochemisch initiiertes Adhäsiv auf Basis von 4-META ergab sowohl im Schmelz- als auch im Dentinbereich einer Kunststoff-Füllung Randspalten (K. Nagata et al, Journal of Biomedical Materials Research 18 (1984) 1089 - 1103). An anderer Stelle wurde als weitere Behinderung bei der Verwendung des 4-META-haltigen Superbonds darauf hingewiesen, daß Schmelz- und Dentinbereiche ein und derselben Kavität mit verschiedenen Ätz- bzw. Reinigungslösungen behandelt werden müssen (Dirass Report E 9 - 4, Biocompatible Materials for the Oral Cavity, Dia Research Institute Inc., 1987).

Mit dem Pyromellithsäure-di-2-methacryloyloxyethylester (PMDM) wurde ein mit dem 4-META verwandtes Monomer zur Verwendung in dentalen Haftvermittlern beschrieben (R.L. Bowen et al, J. Dent. Res. 61, 9 (1982) 1070 - 1076), das ebenfalls in einem Handelsprodukt eingesetzt wird (Tenure von Den Mat). Auch die Anwendung dieses Produktes wird in obiger Literaturstelle als kompliziert beschrieben, da das Dentin vor Applikation des Haftvermittlers in einem zusätzlichen Arbeitsschritt mit einem oberflächenaktiven Comonomer behandelt werden muß. Auch dieses Produkt vermag keine randspaltfreien Füllungen zu gewährleisten.

Es wurde nun gefunden, daß sich mit Hilfe der neuen N-Alkyl-N-(meth)acryloyloxyalkylcarboxamide aromatischer Carbonsäuren und aromatischer Carbonsäureanhydride (I) Adhäsive zur Behandlung der Zahnhartsubstanz formulieren lassen, die bei einfacher Zusammensetzung und einfacher Handhabung randspaltfreie Verklebungen ermöglichen.

Die neuen Verbindungen entsprechen der Formel (I) 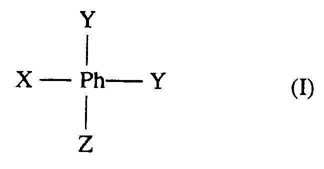
in der
- X: für N-Alkyl-N-(meth)acryloyloxyalkylcarboxamid der Formel

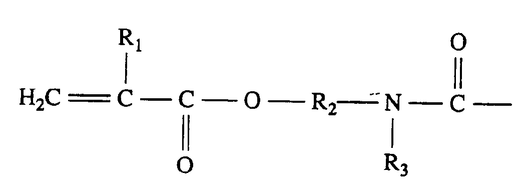

steht, worin
- R₁: Wasserstoff oder Methyl bedeutet,
- R₂: einen zweiwertiger aliphatischer Rest (C₂-C₆) bedeutet und
- R₃: einen einwertiger aliphatischer Rest (C₁-C₄) bedeutet,
- Y: für COOH steht,
wobei nachbarständige Gruppen Y auch zu einer Anhydridgruppe 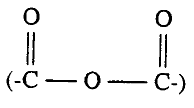 verknüpft sein können,
- Z: für H, X, oder, sofern Y COOH bedeutet, für Y steht und
- Ph: für einen drei- oder vierfach substituierten Benzolring (1,2,3-/1,2,4- oder 1,2,4,5-Substitution) oder einen drei- oder vierfach substituierten Naphthalinring (1,2,6-/1,4,5-/2,3,6-/1,4,5,8- oder 2,3,6,7-Substitution) steht.

(Meth)acryloyl-Derivate im Rahmen der vorliegenden Verbindung sind Derivate der Acrylsäure und der Methacrylsäure.

Die unterschiedlichen Substituenten, Alkyl- und Arylreste der erfindungsgemäßen N-Alkyl-N-(meth)acryloyloxyalkylcarboxamide im Rahmen der allgemeinen Formel (I) haben im allgemeinen die folgende Bedeutung:

Ein zweiwertiger aliphatischer C₂-C₆-Rest (R₂) bedeutet im allgemeinen einen zweiwertigen, geradkettigen oder verzweigten Kohlenwasserstoffrest mit 2 bis 6, bevorzugt 2 oder 3 Kohlenstoffatomen. Beispielsweise seien die folgenden zweiwertigen aliphatischen Reste genannt: Butandiyl, Dimethylethandiyl, Pentandiyl, Neopentandiyl, Hexandiyl, 2,3-Dimethylbutandiyl, Methylethandiyl, Propandiyl, Ethandiyl. Bevorzugt werden als zweiwertige Reste Ethandiyl und Propandiyl.

Ein einwertiger aliphatischer C₁-C₄-Rest (R₃) bedeutet im allgemeinen einen geradkettigen oder verzweigten Kohlenwasserstoffrest mit 1 bis 4, bevorzugt 3 oder 4, Kohlenstoffatomen. Beispielsweise seien die folgenden Alkylreste genannt: Methyl, Ethyl, Propyl, Isopropyl, Butyl, Isobutyl, tert. Butyl. Insbesondere bevorzugt werden tert. Butyl und Isobutyl.
- Z: im Rahmen der allgemeinen Formel (I) bedeutet Wasserstoff (H) oder N-Alkyl-N-(meth)acryloyloxyalkylcarboxamid der Formel 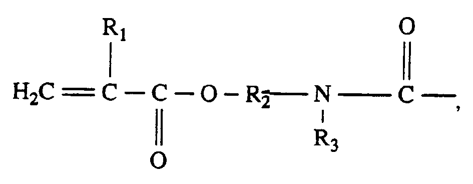 wobei R₁, R₂ und R₃ die angegebenen Bedeutungen haben, oder auch COOH, sofern auch Y die Bedeutung COOH hat.
- Y: kann außer für COOH auch für eine Anhydridgruppe stehen, sofern die beiden enthaltenen Reste Y sich nachbarständig an einem Aromaten gebunden befinden. Als nachbarständig gelten hierbei ortho-Positionen in Benzol- oder Naphthalin-Kernen und die α-Positionen (1,8- bzw. 4,5-Substitution) in Naphthalin-Kernen.
- Ph: bedeutet einen durch X und Y dreifach in 1,2,3- bzw. 1,2,4-Stellung oder durch X, Y und Z vierfach in 1,2,4,5-Stellung substituierten Benzol-Kern oder einen durch X und Y dreifach in 1,2,6-, 1,4,5- bzw. 2,3,6-Stellung oder durch X, Y und Z vierfach in 2,3,6,7- bzw. 1,4,5,8-Stellung substituierten Naphthalin-Kern.

Im Rahmen der Formel (I) bevorzugte Verbindungen entsprechen der Formel (II) 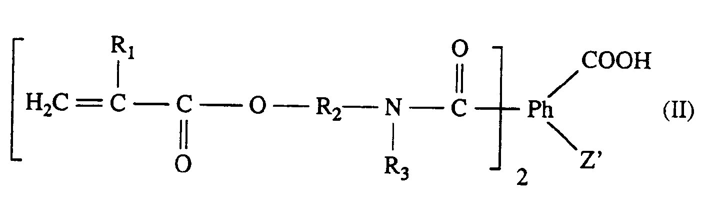
in der
- R₁: Wasserstoff oder Methyl bedeutet,
- R₂: einen zweiwertigen aliphatischen Rest (C₂-C₆) bedeutet,
- R₃: einen einwertigen aliphatischen Rest (C₁-C₄) bedeutet,
- Z': Wasserstoff oder COOH bedeutet

und
- Ph: für einen drei- oder vierfach substituierten Benzolring (1,2,3-/1,2,4- oder 1,2,4,5-Substitution) oder einen drei- oder vierfach substituierten Naphthalinring (1,2,6-/1,4,5-/2,3,6-/1,4,5,8- oder 2,3,6,7-Substitution)

steht.

Eine weitere bevorzugte Gruppe von Verbindungen der Formel (I) entspricht der Formel (III) 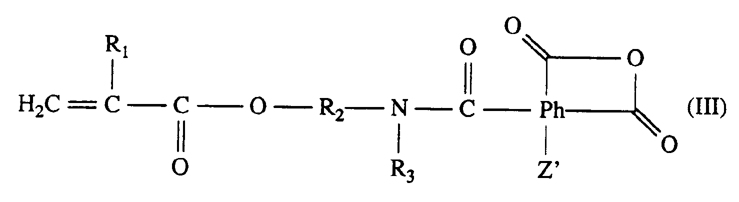
in der
- R₁: Wasserstoff oder Methyl bedeutet,
- R₂: einen zweiwertigen aliphatischen Rest (C₂-C₆) bedeutet,
- R₃: einen einwertigen aliphatischen Rest (C₁-C₄) bedeutet,
- Z': Wasserstoff oder COOH bedeutet

und
- Ph: für einen drei- oder vierfach substituierten Benzolring (1,2,3-/1,2,4- oder 1,2,4,5-Substitution) oder einen drei- oder vierfach substituierten Naphthalinring (1,2,6-/1,4,5-/2,3,6-/1,4,5,8- oder 2,3,6,7-Substitution)

steht.

Eine besonders bevorzugte Gruppe von Verbindungen der Formel (I) entspricht der Formel (IV) 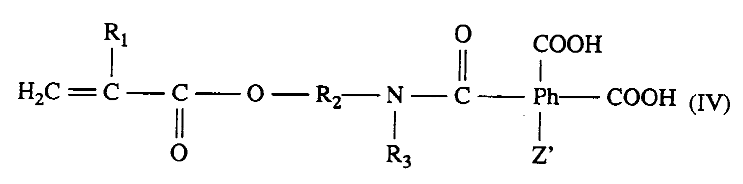
in der
- R₁: Wasserstoff oder Methyl bedeutet,
- R₂: einen zweiwertigen aliphatischen Rest (C₂-C₆) bedeutet,
- R₃: einen einwertigen aliphatischen Rest (C₁-C₄) bedeutet,
- Z': Wasserstoff oder COOH bedeutet

und
- Ph: für einen drei- oder vierfach substituierten Benzolring (1,2,3-/1,2,4- oder 1,2,4,5-Substitution) oder einen drei- oder vierfach substituierten Naphthalinring (1,2,6-/1,4,5-/2,3,6-/1,4,5,8- oder 2,3,6,7-Substitution)

steht.

Im einzelnen seien beispielsweise die folgenden N-Alkyl-N-(meth)acryloyloxyalkylcarboxamide genannt:

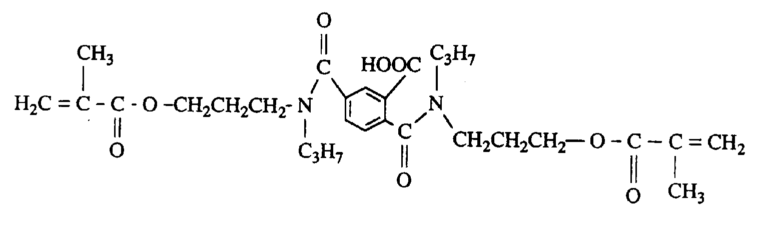

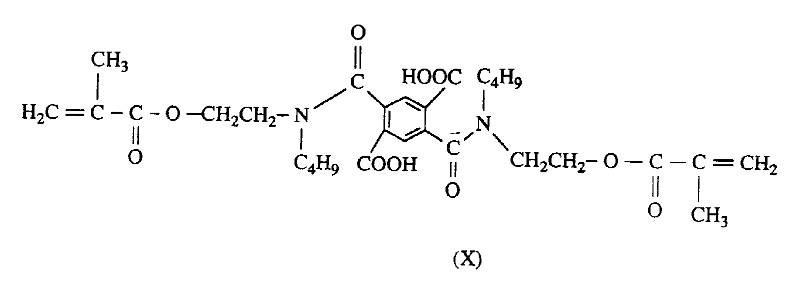

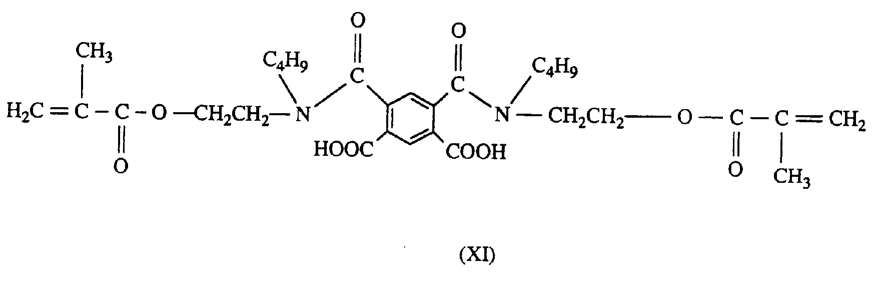

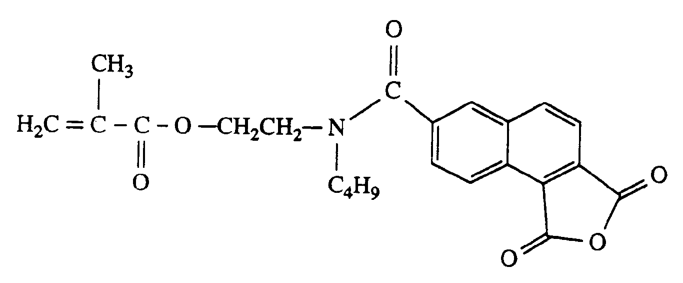

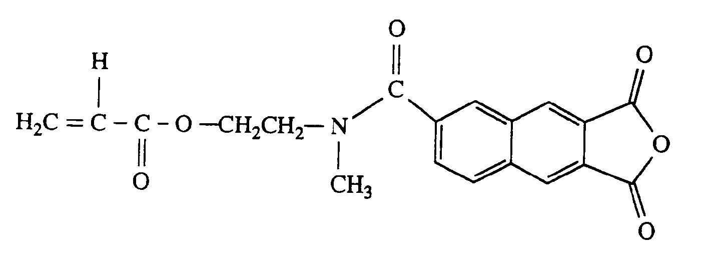

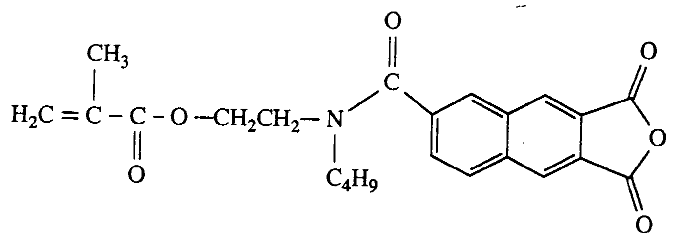

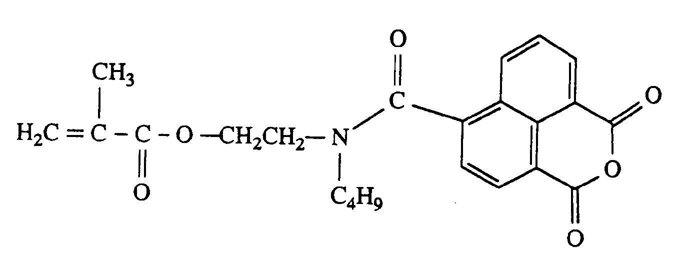

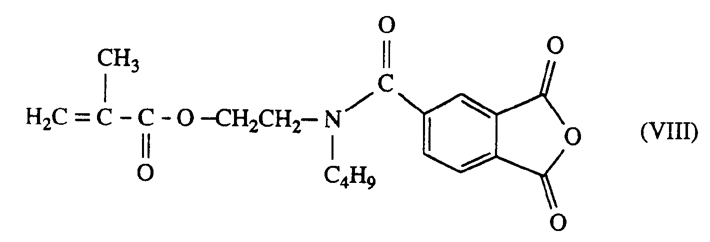

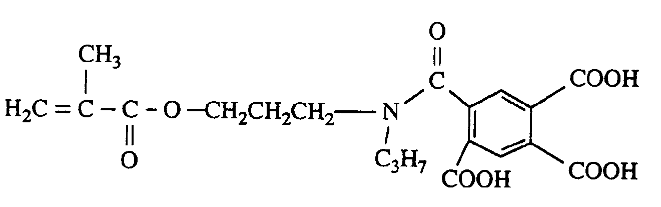

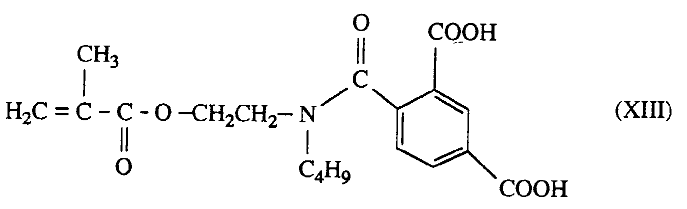

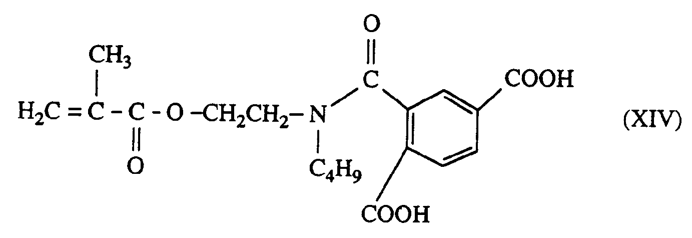

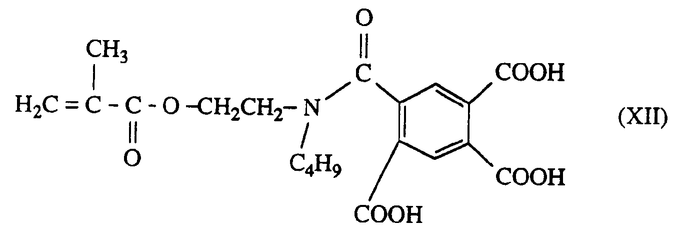

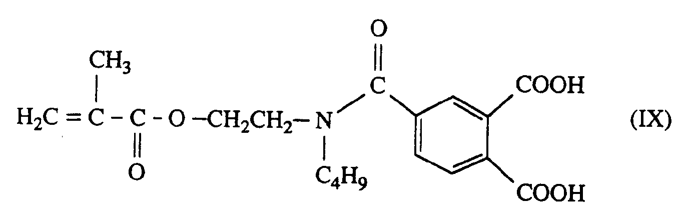

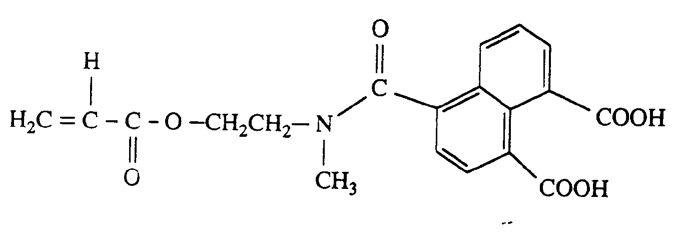

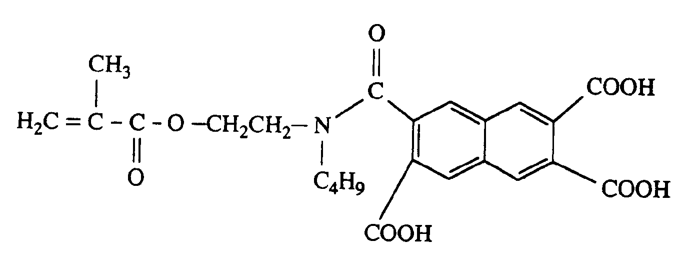

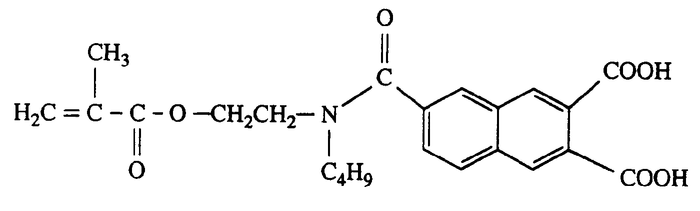

Es wurde weiterhin ein Verfahren zur Herstellung von N-Alkyl-N(meth)acryloyloxyalkylcarboxamiden aromatischer Carbonsäuren und aromatischer Carbonsäureanhydride der Formel (I) gefunden, das dadurch gekennzeichnet ist, daß man

Monoanhydride der Formel (V) 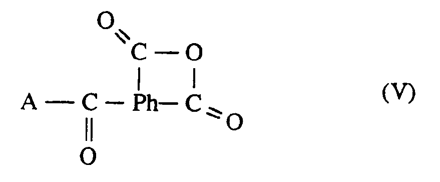
oder Dianhydride der Formel (VI) 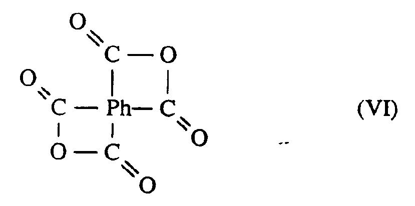
worin
- A: Hydroxy (OH) oder Chlor (Cl) bedeutet und
- Ph: für einen drei- oder vierfach substituierten Benzolring (1,2,3-/1,2,4- oder 1,2,4,5-Substitution) oder einen drei- oder vierfach substituierten Naphthalinring (1,2,6- 1,4,5- 2,3,6- 1,4,5,8- oder 2,3,6,7-Substitution)

steht
in einem organischen Lösungsmittel zwischen -30 und 110°C gegebenenfalls in Gegenwart einer Base
mit einem Amin der (VII) 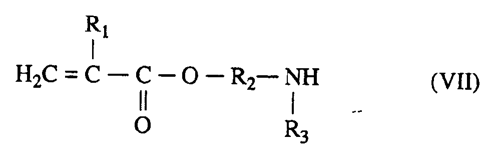
in der
- R₁: Wasserstoff oder Methyl bedeutet,
- R₂: einen zweiwertiger aliphatischer Rest (C₂-C₆) bedeutet und
- R₃: einen einwertiger aliphatischer Rest (C₁-C₄) bedeutet,

umsetzt und
gegebenenfalls das erhaltene Produkt mit Wasser zwischen 5 und 100°C hydrolysiert.

Als Monoanhydride der Formel (V) werden in das erfindungsgemäße Verfahren bevorzugt die käuflichen Trimellitsäure-Derivate 1,2,4-Benzoltricarbonsäureanhydridchlorid und 1,2,4-Benzoltricarbonsäureanhydrid, die literaturbekannten Hemimellitsäure-Derivate 1,2,3-Benzoltricarbonsäureanhydrid (Beilstein Band H/18, S. 468, 4. Aufl. Springer Verlag 1934) und 1,2,3-Benzoltricarbonsäureanhydridchlorid (US 3, 920, 667) bzw. die von Harashima et. al. (Dental Materials Journal 7 (1988) 142) hergestellten Naphthalintricarbonsäure-Derivate 1,2,6-, 2,3,6- und 1,4,5-Naphthalintricarbonsäureanhydridchlorid eingesetzt.

Als Dianhydride der Formel (VI) seien bevorzugt die käuflichen Verbindungen Benzol-1,2,4,5-tetracarbonsäuredianhydrid (Pyromellitsäuredianhydrid) und Naphthalin-1,4,5,8-tetracarbonsäuredianhydrid bzw. das Naphthalin-2,3,6,7-tetracarbonsäuredianhydrid, welches sich einfach durch Dehydratisierung aus der bekannten Naphthalin-2,3,6,7-tetracarbonsäure (Chiba et al, Chem. Lett. 11 (1988) 1933 - 1936) bildet, genannt.

Aminoalkyl(meth)acrylsäureester der Formel (VII) sind handelsüblich oder können in bekannter Weise durch Veresterung von Alkanolaminen gegebenenfalls unter Verwendung von Schutzgruppen für die Aminofunktion hergestellt werden.

Geeignete organische Lösungsmittel für das erfindungsgemäße Verfahren sind aprotische Lösungsmittel wie Dioxan, Tetrahydrofuran, N,N-Dimethylformamid, N,N-Dimethylacetamid, Dimethylsulfonamid und Aceton. Bevorzugt geeignet sind Toluol und Diethylether. Besonders bevorzugt sind Xylol, Methylenchlorid, Chloroform und Methyl-tert.butylether.

Ein geeigneter Temperaturbereich zur Umsetzung des Amines VII liegt zwischen -30 und 110°C. Bevorzugt wird diese Reaktion zwischen -10 und 50°C und besonders bevorzugt zwischen -5 und 30°C durchgeführt. Beim erfindungsgemäßen Verfahren können zusätzlich anorganische oder organische Basen verwendet werden.

Bevorzugte anorganische Basen sind die schwach basischen Carbonate und Hydrogencarbonate des Natrium und Kaliums. Bevorzugte organische Basen sind tert. Amine, wobei Triethylamin und Pyridin insbesondere bevorzugt sind. Die Basen werden bezüglich des eingesetzten Anhydrides der Formel (V) bzw. (VI) in einer äquimolaren bis fünffach molaren Menge eingesetzt, wobei ein 2- bis 3-facher molarer Überschuß bevorzugt ist. Die organischen Basen wirken auf diese Weise zusätzlich lösungsvermittelnd. Es kann allerdings auch vorteilhaft sein, das erfindungsgemäße Verfahren in einer übersättigten Lösung des Anhydrides (V) bzw. (VI), d.h. in einer Dispersion durchzuführen, da das Reaktionsprodukt besser löslich ist als das Anhydrid und infolgedessen das Anhydrid mit fortschreitender Reaktion gelöst wird. Zur Herstellung der Derivate mit benachbarten Carbonsäuregruppen der allgemeinen Formel (I) werden die im obigen Prozeß erhaltenen Anhydride bei Temperaturen zwischen 5 und 100°C und bevorzugt zwischen 20 und 50°C hydrolysiert. Dies kann sowohl nach Isolierung der Anhydride der allgemeinen Formel (I) geschehen, oder auch ohne Isolierung durch direkte Hydrolyse des Reaktionsansatzes. Zur Durchführung dieser Hydrolyse wird Wasser in äquimolarer, bevorzugt jedoch in über zehnfach molarer Menge zugegeben. Es ist ein besonderes Kennzeichen des erfindungsgemäßen Verfahrens, daß es vorteilhaft sein kann, daß das zugesetzte Wasser mit dem verwendeten Lösungsmittel der Reaktion nicht mischbar sein muß. Gerade die Hydrolyse im Zweiphasengemisch ist bevorzugt, da hierbei gezielt die reaktivste Anhydridgruppe, nicht jedoch die Amid- und Esterbindungen in der erfindungsgemäßen Verbindung (I) angegriffen wird.

In einer besonderen Ausführungsform des erfindungsgemäßen Verfahrens kann das Wasser auch in einer geringeren als der äquimolaren Menge zugesetzt werden bzw. auf die Wasserzugabe auch ganz verzichtet werden. Dies ist dann der Fall, wenn zuvor Carbonate oder Hydrogencarbonate bei der Reaktion zwischen Anhydriden (V) bzw. (VI) und dem Amin (VII) verwendet wurden. Im Verlaufe dieser Reaktion setzen die Carbonate und Hydrogencarbonate Reaktionswasser frei, das die beabsichtigte Hydrolyse der Anhydridgruppe bedingt.

Die hier beschriebene Hydrolyse kann zudem durch gezielte Zugabe von Säuren, vor allem Schwefelsäure, Phosphorsäure, Toluolsulfonsäure oder sauren Ionenaustauschern, bzw. durch Zugabe von Basen wie Natrium- und Kalium-Hydroxid, -Carbonat oder -Hydrogencarbonat katalysiert werden.

Zur Veranschaulichung des erfindungsgemäßen Verfahrens sei im folgenden eine allgemeine Synthese beschrieben. Zu einer Lösung aus Anhydrid (V) und einem Basen-Überschuß in einem organischen Lösungsmittel wird bei Raumtemperatur das Amin (VII) getropft und anschließend bei erhöhter Temperatur so lange gerührt, bis das Amin (VII) laut IR-Kontrolle verbraucht ist. Es wird dann Wasser im Überschuß zugegeben und die entsprechende Mischung bei erhöhter Temperatur so lange kräftig durchgerührt, bis im IR-Spektrum des Ansatzes keine Anhydridbanden mehr sichtbar sind. Das entstandene erfindungsgemäße Produkt läßt sich als Anion in eine alkalische wäßrige Phase extrahieren und aus dieser nach Ansäuern gewinnen.

Die nach Hydrolyse erhaltene aromatische Carbonsäure läßt sich für den Fall, daß sie nachbarständige Carbonsäuregruppen enthält, auch durch Erhitzen mit Essigsäureanhydrid in einem organischen, aprotischen Lösungsmittel in das entsprechende Anhydrid zurückführen. Die Anhydride werden auf diese Weise in besonders guter Reinheit erhalten.

Die erfindungsgemäßen Zubereitungen der neuen N-Alkyl-N-(meth)acryloyloxyalkylcarboxamide (I) enthalten neben diesen Carboxamiden ein Lösungsmittel und gegebenenfalls Initiatoren, Coaktivatoren und (Meth)acrylsäureester, die Vernetzungen ausbilden können. Insbesondere können auch Gemische verschiedener erfindungsgemäßer Carboxamide (I) in den erfindungsgemäßen Zubereitungen eingesetzt werden.

Die Lösungsmittel im Rahmen der erfindungsgemäßen Zubereitungen sollen die Komponenten lösen und sollen, durch die Anwendung bedingt, untoxisch sein. Bevorzugt seien Wasser und flüchtige organische Lösungsmittel wie Methanol, Ethanol, Propanol, Isopropanol, Aceton, Methylethylketon, Essigsäuremethyl- oder -ethylester bzw. Tetrahydrofuran genannt. Im allgemeinen setzt man 10 bis 1000 Gew.-Teile, bevorzugt 50 bis 300 Gew.-Teile des Lösungsmittels, bezogen auf die Carboxamide (I) ein. Insbesondere bevorzugt können auch Mischungen dieser Lösungsmittel sein, wobei wäßrige Mischungen ganz besonders bevorzugt sind.

Initiatoren im Rahmen der vorliegenden Erfindung sind Radikalbildner, die eine radikalische Polymerisation auslösen. Bevorzugt sind Fotoinitiatoren, die unter der Einwirkung von Licht, beispielsweise UV-Licht, sichtbarem Licht oder Laserlicht, eine radikalische Polymerisation auslösen.

Die sogenannten Fotopolymerisationsinitiatoren sind an sich bekannt (Houben Weyl, Methoden der organischen Chemie, Band E 20, Seite 80 ff, Georg Thieme Verlag Stuttgart, 1987). Vorzugsweise handelt es sich um Mono- oder Dicarbonylverbindungen, wie Benzoin und dessen Derivate, insbesondere Benzoinmethylether, Benzil und Benzilderivate, beispielsweise 4,4-Oxidibenzil und andere Dicarbonylverbindungen wie Diacetyl, 2,3-Pentandion und α-Diketoderivate des Norbornans und substituierter Norbornane, Metallcarbonyle wie Penta-carbonylmangan oder Chinone wie 9,10-Phenanthrenchinon und Naphthochinon. Besonders bevorzugt ist Campherchinon.

Die erfindungsgemäßen Zubereitungen enthalten im allgemeinen 0,01 bis 2 Gew.-Teile, bevorzugt 0,1 bis 0,5 Gew.-Teile des Initiators, bezogen auf 1 Gew.-Teil des Carboxamids (I).

Enthält eines der mit der erfindungsgemäßen Adhäsivkomponente im Kontakt stehenden Fügeteile bereits einen Initiator der beschriebenen Art, kann auf den Initiator in der Adhäsivkomponente auch ganz verzichtet werden.

Es kann vorteilhaft sein, den erfindungsgemäßen Zubereitungen Coaktivatoren zuzusetzen, die die Polymerisationsreaktion beschleunigen. Bekannte Beschleuniger sind beispielsweise Amine wie p-Toluidin, Dimethyl-p-toluidin, Trialkylamine wie Trihexylamin, Polyamine wie N,N,N',N'-Tetraalkylalkylendiamin, Barbitursäure und Dialkylbarbitursäure. Besonders bevorzugt sind Dimethylaminobenzolsulfonamide entsprechend DE-A-31 35 113.

Die Coaktivatoren werden im allgemeinen in einer Menge von 0,02 bis 4 Gew.-%, bevorzugt 0,2 bis 1 Gew.-%, bezogen auf die Menge an polymerisierbaren Verbindungen, eingesetzt.

Als weitere Komponenten für die erfindungsgemäßen Zusammensetzungen kommen (Meth)-acrylsäureester, die Vernetzungen ausbilden können, in Frage. (Meth)acrylsäureester, die Vernetzungen ausbilden können, enthalten im allgemeinen 2 oder mehr polymerisierbare aktive Doppelbindungen im Molekül. Bevorzugt seien Ester der (Meth)-acrylsäure mit 2- bis 5-wertigen Alkoholen mit 2 bis 30 Kohlenstoffatomen genannt. Besonders bevorzugt werden Epoxid(meth)acrylate und Urethan(meth)acrylate.

Beispielsweise seien (Meth)-acrylsäureester der Formel 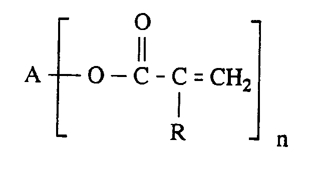
in der
- A: einen geradkettigen, verzweigten, cyclischen, aliphatischen, aromatischen oder gemischt aliphatisch-aromatischen Rest mit 2 bis 25 C-Atomen, der durch -O- oder NH-Brücken unterbrochen und mit Hydroxy, Oxy, Carboxy, Amino oder Halogen substituiert sein kann,

bedeutet,
- R: H oder Methyl bedeutet und
- n: für eine ganze Zahl von 2 bis 8, vorzugsweise 2 bis 4, steht,

genannt.

Vorzugsweise seien Verbindungen der folgenden Formeln genannt: 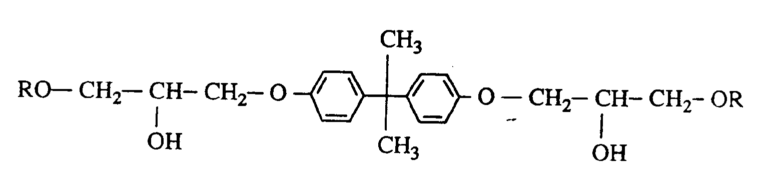 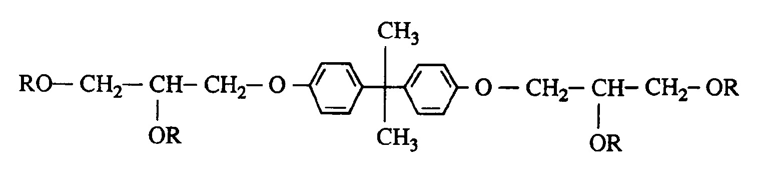 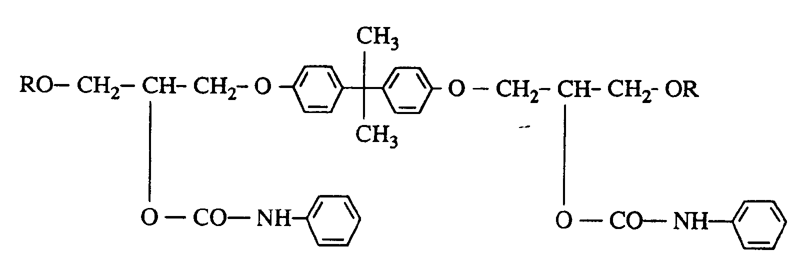 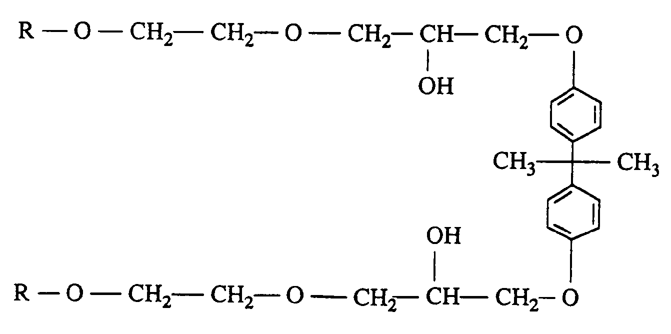 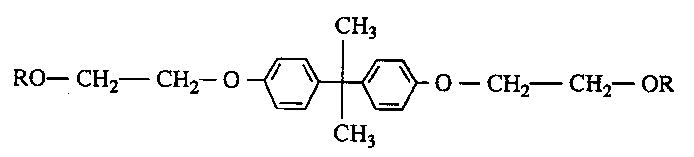 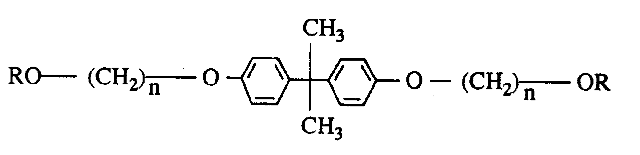 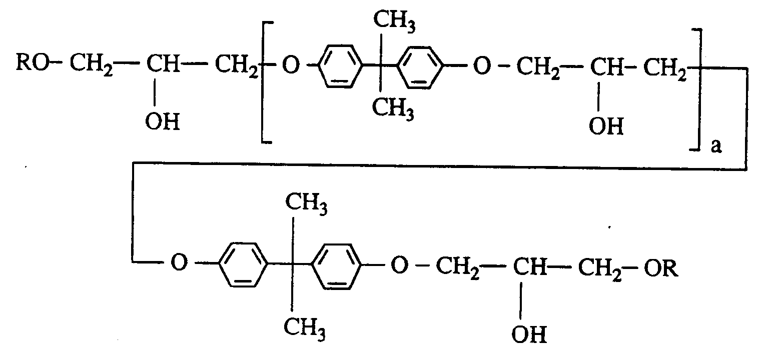
wobei a eine Zahl von 1 bis 4 bedeuted 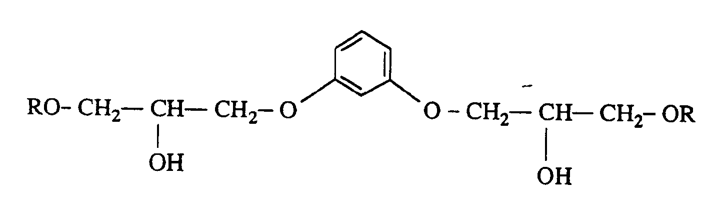 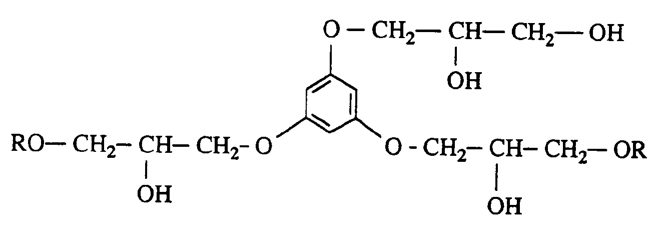 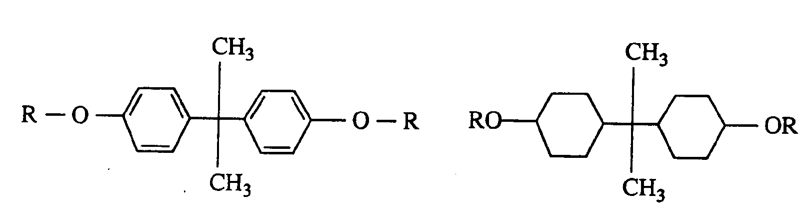 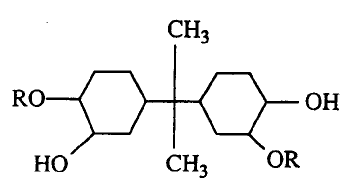 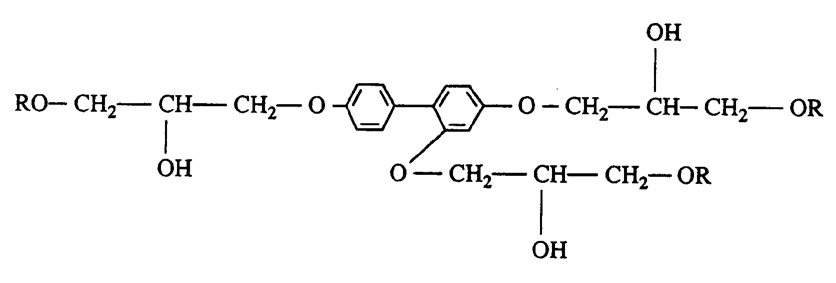 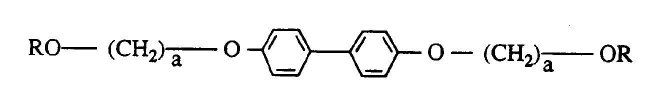
wobei a eine Zahl von 1 bis 4 bedeuted, 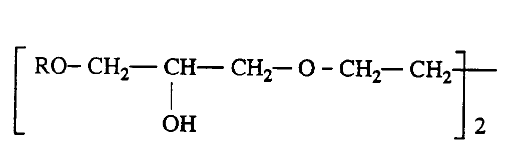 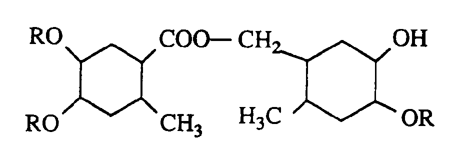 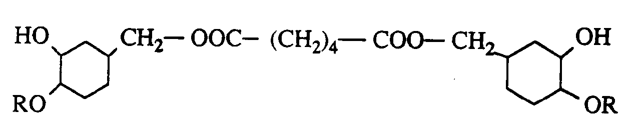 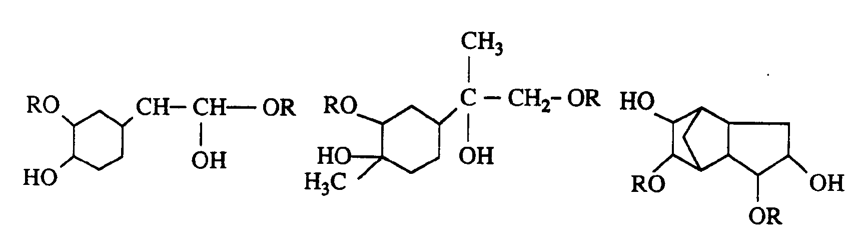 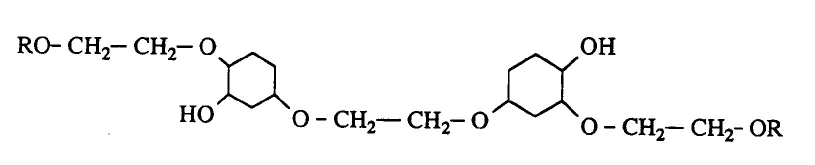 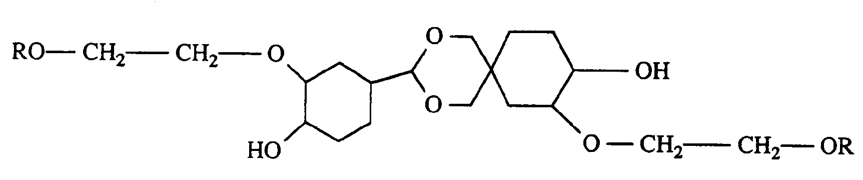 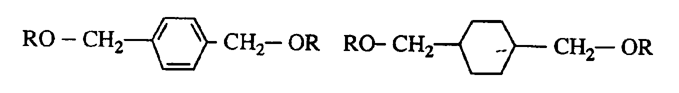 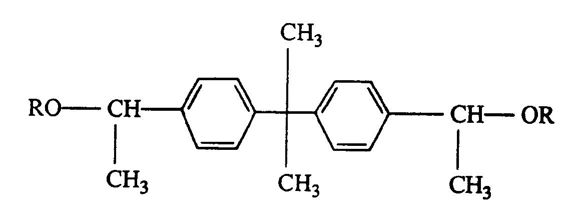 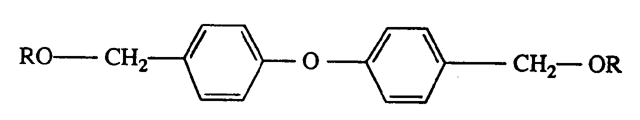 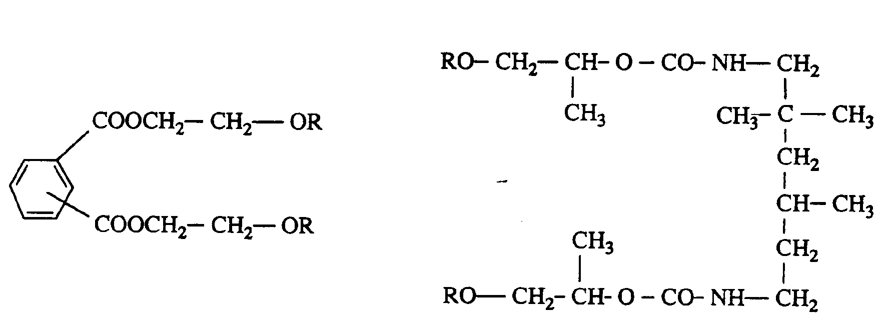
in der ortho-, meta- oder para-Form 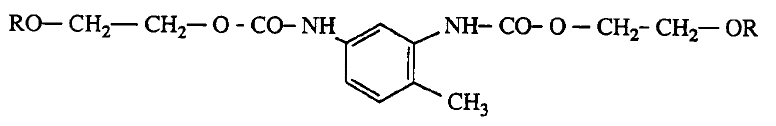 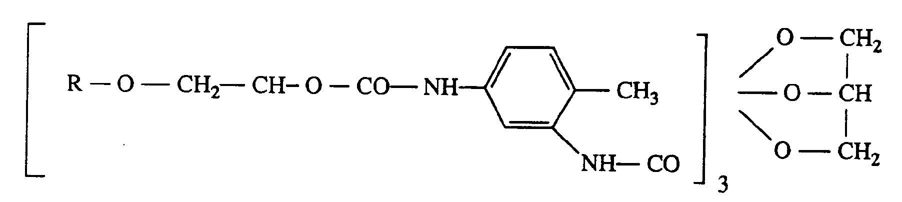
worin R für 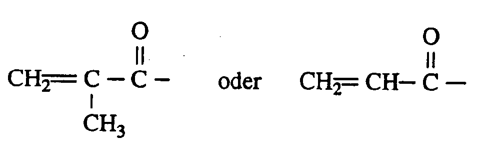
steht.

Außerdem seien Derivate des Tricyclodecans (EP-A 0 023 686) und Umsetzungsprodukte aus Polyolen, Diisocyanaten und Hydroxyalkylmethacrylaten (DE-A 37 03 120, DE-A 37 03 080 und DE-A 37 03 130) genannt. Beispielsweise seien die folgenden Monomeren genannt: 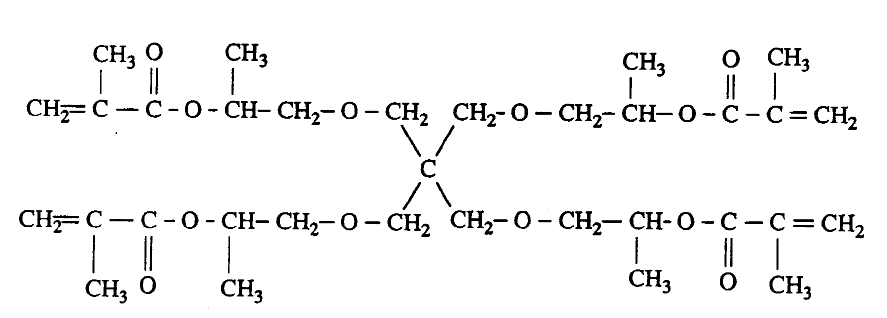 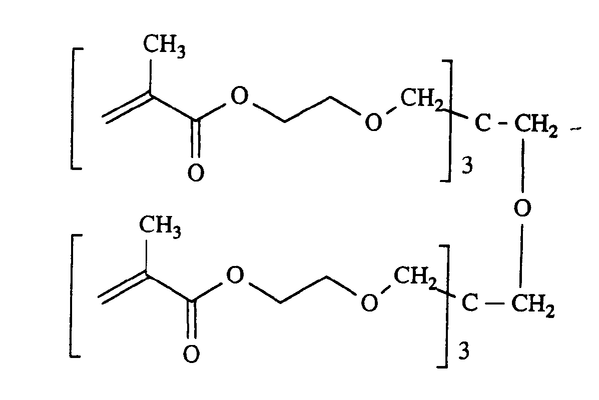 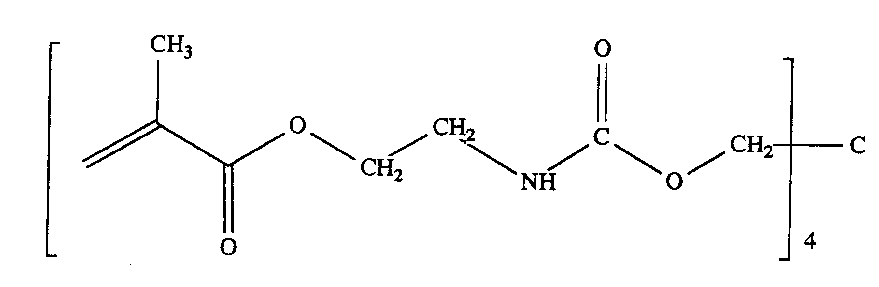 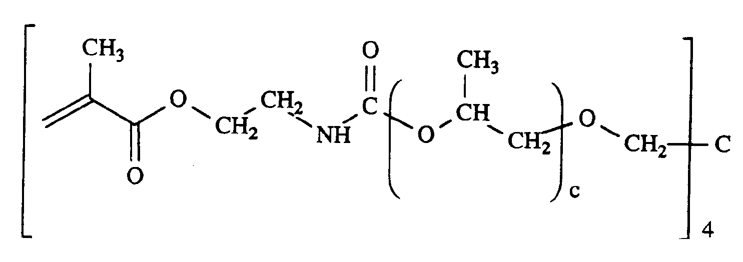
c=1.225 (statistischer Mittelwert für 4 Ketten) 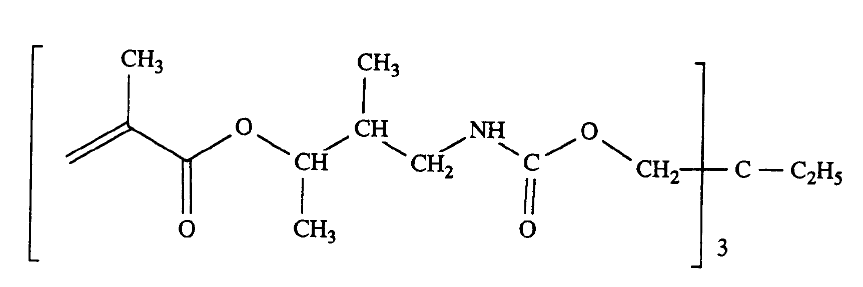 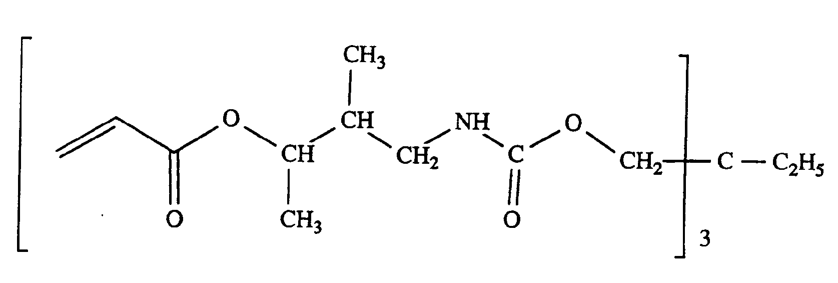 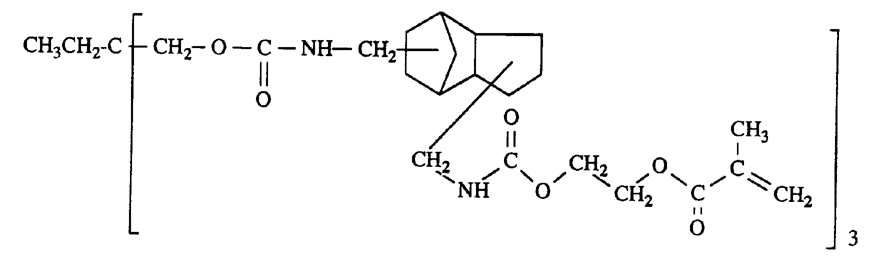 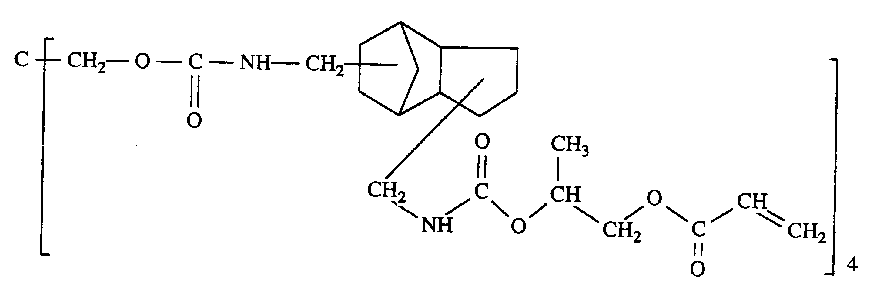 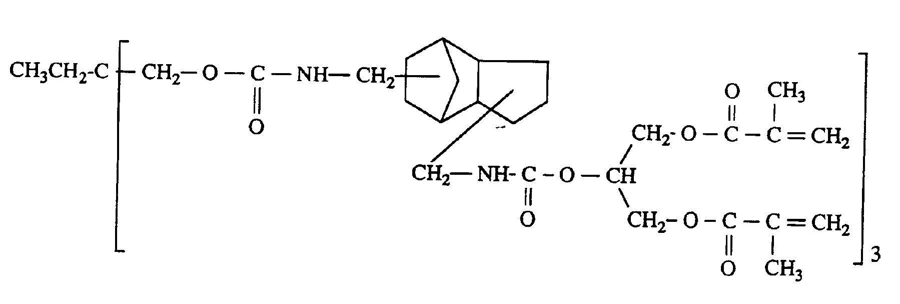 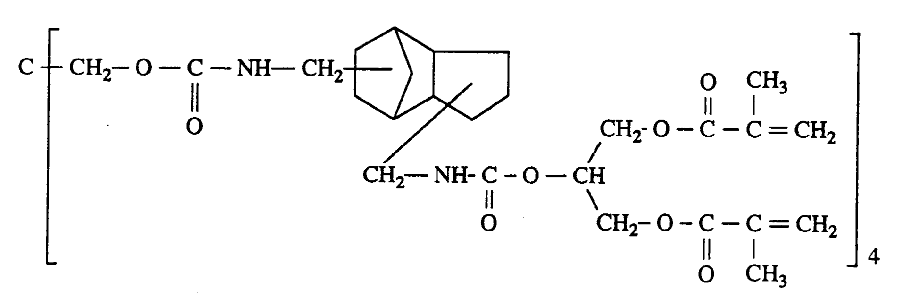 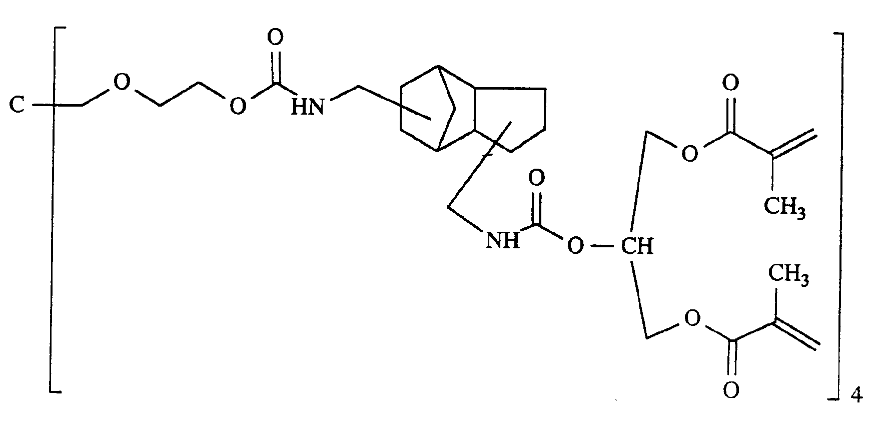 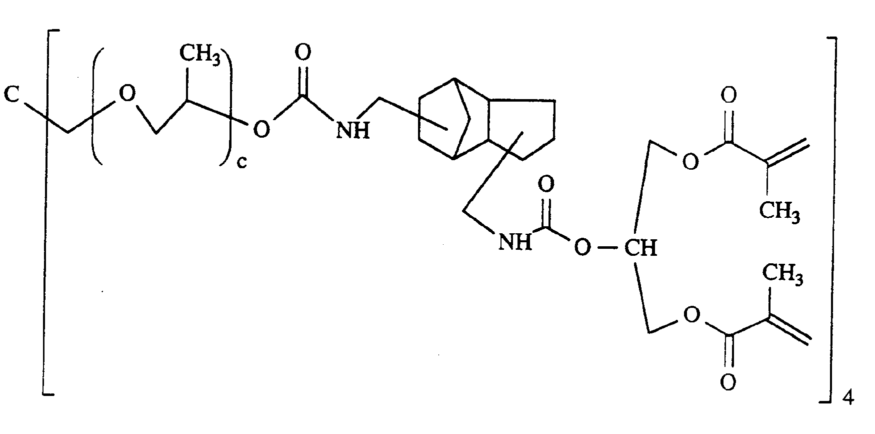
c=1.225 (statistischer Mittelwert für 4 Ketten) 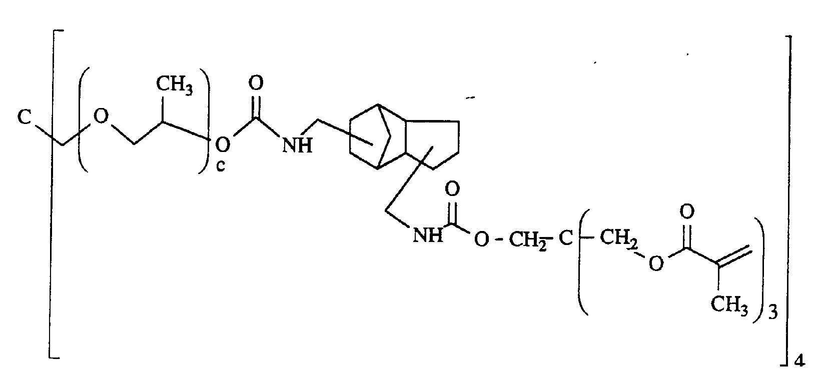
c=1.225 (statistischer Mittelwert für 4 Ketten) 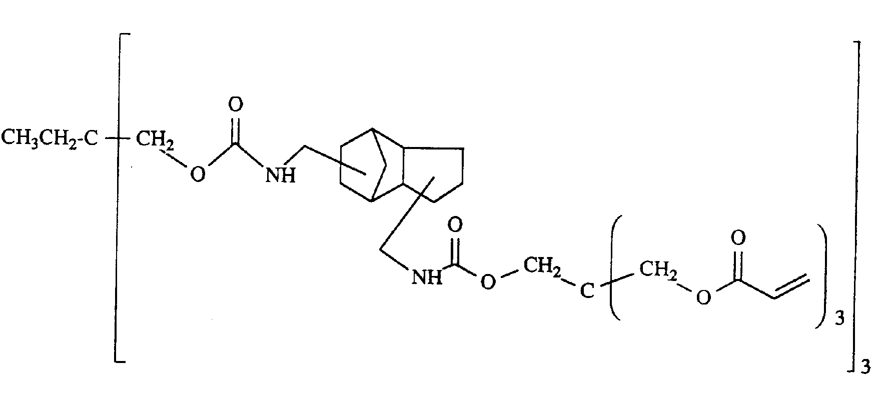 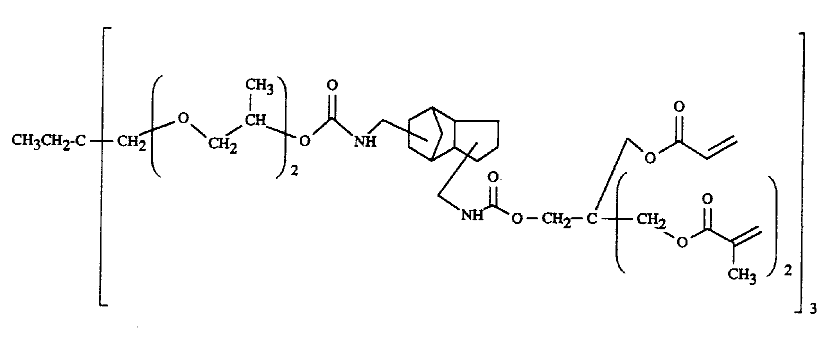 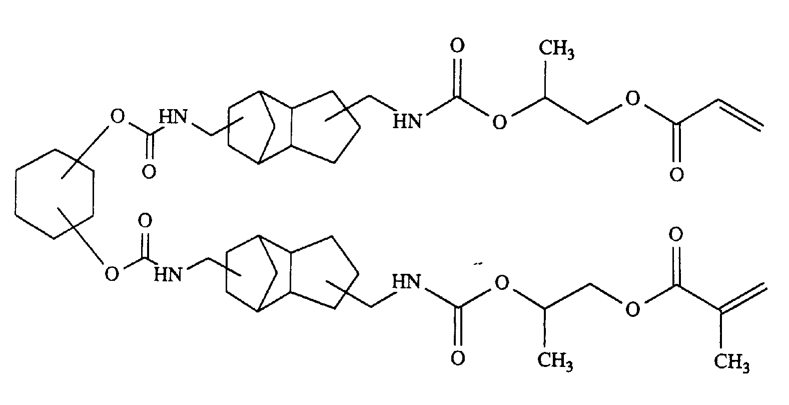 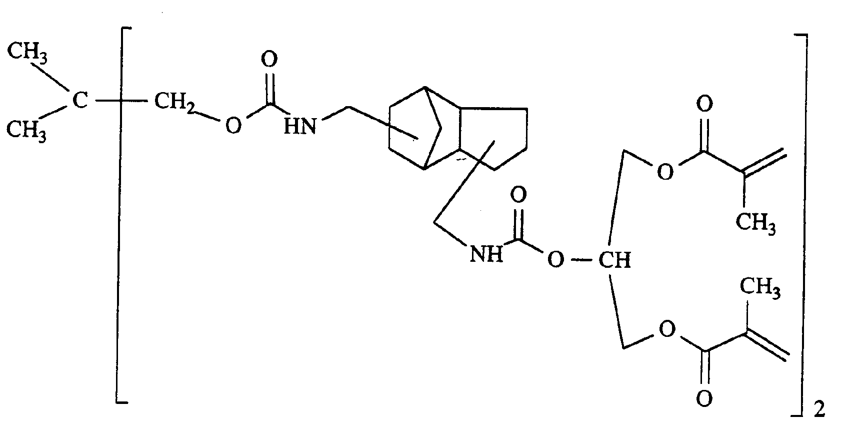 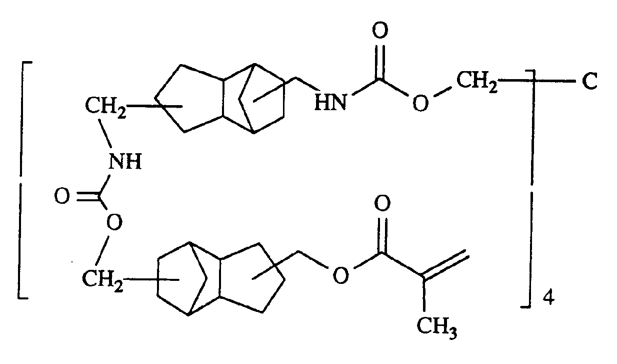 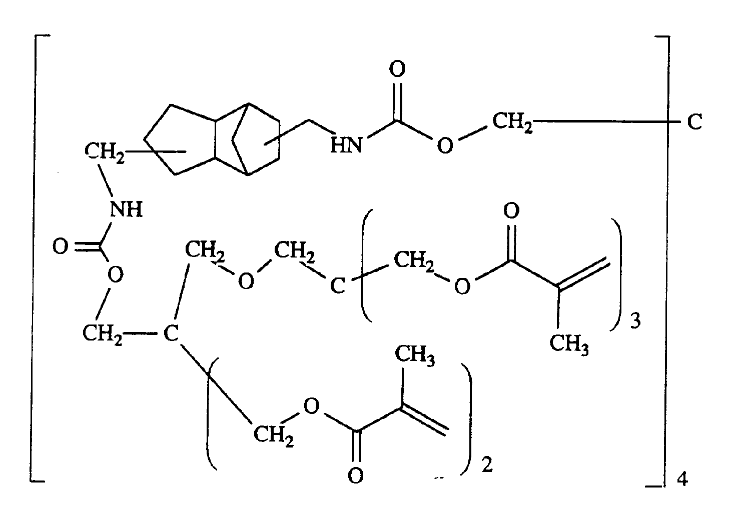 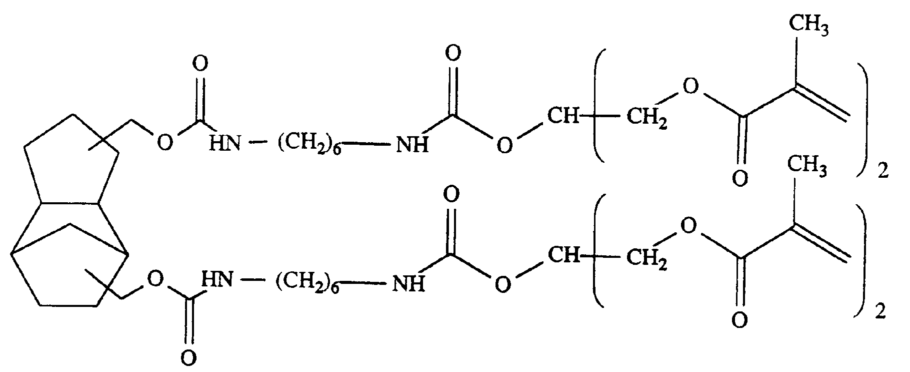 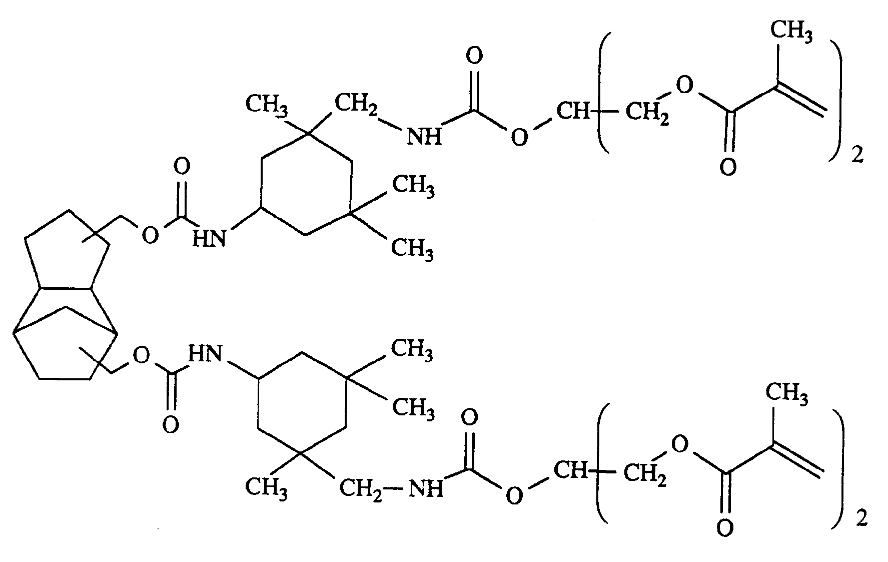 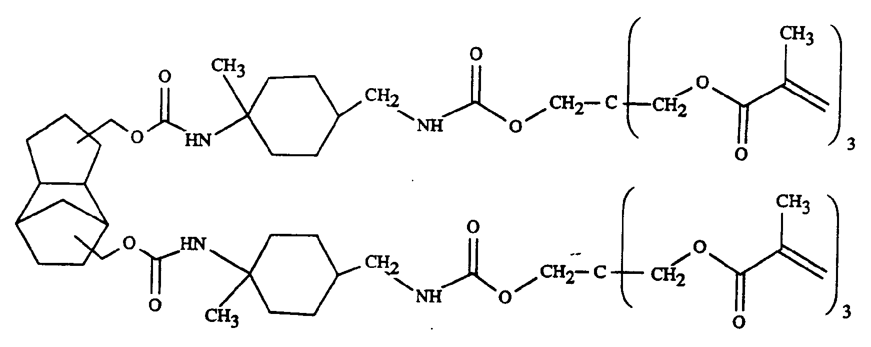 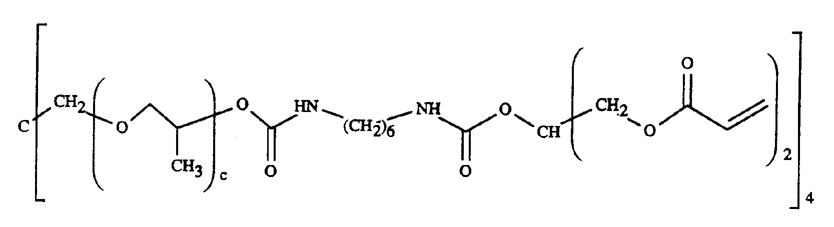
c=1.225 (statistischer Mittelwert für 4 Ketten) 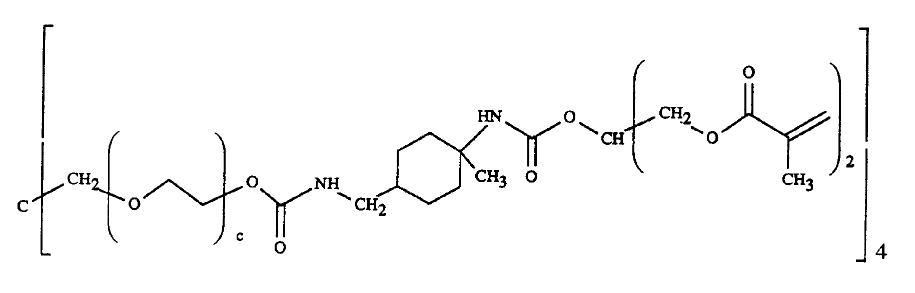
c=1.225 (statistischer Mittelwert für 4 Ketten)

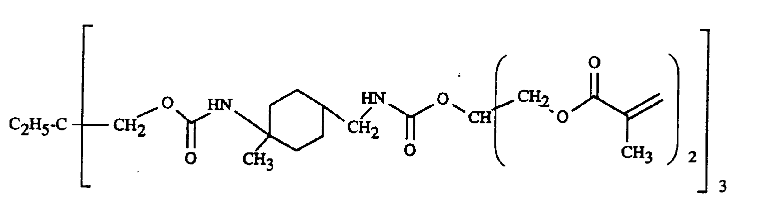

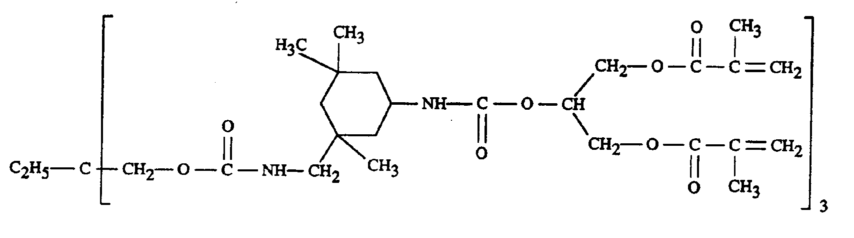

Als Methacrylsäureester besonders bevorzugt wird das sogenannte Bis-GMA der Formel

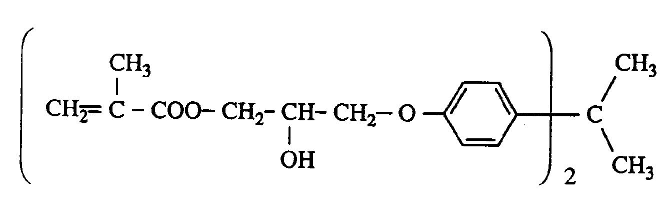

Selbstverständlich ist es möglich, Mischungen der verschiedenen (Meth)acrylsäureester, die Vernetzungen ausbilden können, einzusetzen. Beispielsweise seien Mischungen von 20 bis 70 Gew.Teilen Bis-GMA und 30 bis 80 Gew.-Teilen Triethylenglykoldimethacrylat genannt.

Die Adhäsivkomponenten gemäß dieser Erfindung können weiterhin bis zu 10 Gew.-Teilen üblicher Zusätze wie Stabilisatoren, Inhibitoren und Lichtschutzmittel enthalten. Der erfindungsgemäßen Zubereitungen können hergestellt werden, indem man das Carboxamid (I), das Lösungsmittel und den Initiator und gegebenenfalls die anderen Komponenten durch kräftiges Rühren mischt.

Die erfindungsgemäßen Zubereitungen können als Adhäsivkomponente zur Behandlung von Zahnhartsubstanz und als Klebemittel in Knochenzement verwendet werden.

In einer besonderen Ausführungsform konditioniert man das kollagenhaltige Material vor der Behandlung mit der erfindungsgemäßen Zubereitung mit einer Flüssigkeit mit einem pH-Wert im Bereich von 0,1 bis 3,5. Diese enthält im allgemeinen Säuren mit einem pKₛ-Wert kleiner als 5 und gegebenenfalls eine amphotere Aminoverbindung mit einem pKₛ-Wert im Bereich von 9,0 bis 10,6 und einem pK_{B}-Wert im Bereich von 11,5 bis 12,5. Folgende Säuren können z.B. in der Konditionierflüssigkeit enthalten sein: Phosphorsäure, Salpetersäure, Brenztraubensäure, Zitronensäure, Oxalsäure, Ethylendiamintetraessigsäure, Essigsäure, Weinsäure, Äpfelsäure. Als amphotere Aminoverbindungen seien vorzugsweise Verbindungen der Formel 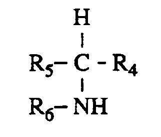
in der
- R₄: für eine Carboxylgruppe steht,
- R₅: Wasserstoff, gegebenenfalls durch Hydroxy, Thio, Methylthio, Carboxy, Amino, Phenyl, Hydroxy-phenyl oder die Gruppen

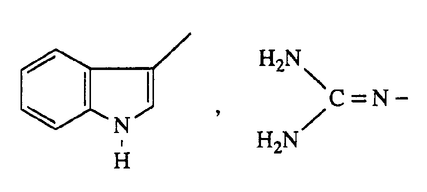

substituierter Niederalkylrest,
- R₆: Wasserstoff oder Phenyl bedeutet und

wobei die Reste R₄ und R₆ durch einen Propylrest verbunden sein können, oder in der
- R₄: für Wasserstoff steht,
- R₅: für die Gruppe

― B ―NH₃D

in der
- B: für einen zweibindigen Alkylenrest mit 1 bis 6 Kohlenstoffatomen und
- D: für Halogen steht, und
- R₆: Wasserstoff bedeutet, genannt.

Beispielsweise seien die folgenden amphoteren Aminoverbindungen genannt:

Glycin, Serin, Threonin, Cystein, Thyrosin, Asparagin, Glutamin, Alanin, Valin, Leucin, Isoleucin, Prolin, Methionin, Phenylalanin, Tryptophan, Lysin, Arginin, Histidin, N-Phenylglycin, Ethylendiaminhydrochlorid, Ethylendiaminhydrobromid, Propylendiaminhydrochlorid, Propylendiaminhydrobromid, Butylendiaminhydrochlorid, Butylendiaminhydrobromid, Leucinhydrochlorid und Histidinhydrochlorid. Weiterhin kann die Konditionierflüssigkeit Stoffe aus der Gruppe der Polyethylenglykole und Metallhydroxide enthalten. Insbesondere können die oben aufgeführten mehrbasigen Säuren auch als teilweise Metallsalze eingesetzt werden, solange freie Säurefunktionen verbleiben.

Konditionierflüssigkeiten, die mindestens eine der Säuren aus der Gruppe der Brenztraubensäure, Ethylendiamintetraessigsäure und Zitronensäure sowie gegebenenfalls eine amphotere Aminoverbindung aus der Gruppe des Glycins, N-Phenylglycins und Prolins enthalten, sind besonders bevorzugt.

Die Anwendung der erfindungsgemäßen Zubereitungen kann beispielsweise wie folgt durchgeführt werden:

Bei einer Zahnreperatur trägt man beispielsweise nach einer mechanischen Reinigung des kollagenhaltigen Zahnmaterials zuerst die Konditionierflüssigkeit mit etwas Warte auf, läßt eine kurze Zeit (beispielsweise 60 Sekunden) einwirken, spült das Zahnmaterial mit Wasser und trocknet es im Luftstrom. Dann trägt man die erfindungsgemäße Zubereitung beispielsweise mit einem Kleinen Pinsel in einer dünnen Schicht auf und trocknet im Luftstrom. Nach der erfindungsgemäßen Behandlung trägt man die eigentliche Füllmasse, beispielsweise im Dentalbereich übliche Kunststoffüllungsmassen (K. Eichner, "Zahnärztliche Werkstoffe und ihre Verarbeitung", Bd. 2, S. 135ff, Hüthig Verlag, 5. Aufl. 1985) auf.

### Beispiele 1 bis 5 (Herstellung erfindungsgemäßer Carboxamide)

### Beispiel 1:

Trimellitsäure-N-tert.butyl-N-methacryloyloxyethyl-amidanhydrid (VIII)

Zu einer Lösung aus 210.57 g (1.000 mol) Benzoltricarbonsäureanhydridchlorid in 650 ml trockenem Dichlormethan und 202,38 g (2.000 mol) trockenem Triethylamin wurde bei -5°C unter Rühren eine Lösung von 185.27 g (1.000 mol) N-tert.-Butyl-2-aminoethylmethacrylat in 250 ml trockenem Dichlormethan getropft. Nach dreistündigem Rühren bei Raumtemperatur wurde der ausgefallene helle Feststoff abgesaugt, das Filtrat wäßrig extrahiert und getrocknet.

Die erhaltene Dichlormethan-Lösung enthielt das gewünschte Produkt (VIII) und konnte direkt zur Hydrolyse eingesetzt werden.

Nach Zusatz von 200 mg 2,6-Di-tert.butylkresol läßt sich die erhaltene Lösung zu 280,31 g (78 % d. Th.) eines gelblichen Feststoffes an Trimellitsäure-N-tert.-butyl-N-methacryloyloxyethylamidanhydrid (VIII) einengen.

Schmelzpunkt: 58°C
- IR (KBr):: ν = 2960, 1860, 1783, 1720, 1640, 1380, 1240, 1195, 1150, 930, 889 cm⁻¹.
- ¹H-NMR: (CDCl₃, 200 MHz): δ = 1,59 (s, 9 H, C(CH₃)₃), 1,87 (bs, 3 H, = CCH₃), 3,65 (t, J = 6,3 Hz, 2 H, NCH₂), 4,16 (t, J = 6,3 Hz, 2 H, OCH₂), 5,64, 6,03(2 bs, je 1 H, vinyl.-H), 7,95 (m, 3 H, arom. H) ppm.

MS (70eV): m/z = 344 (M-CH₃), 273 (M-C₃H₅COOH), 217 (M-C₃H₅COOH-C₄H₈) 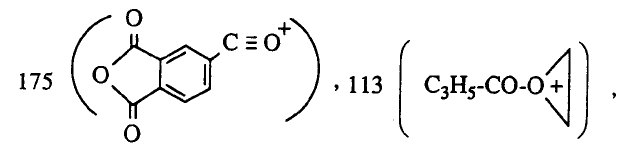
69 (C₃H₅-C≡O⁺), 57' (C₄H₉⁺).

### Beispiel 2:

Trimellitsäure-N-tert.butyl-N-methacryloyloxyethylamid (IX)

700 ml der Dichlormethanlösung aus Beispiel 1 enthaltend 218.03 g (0.607 mol) des Anhydrides (VIII) wurden mit 200 ml Wasser versetzt und 22 Stunden bei 41°C unter kräftigem Rühren zum Rückfluß erhitzt. Nach Trocknen und Stabilisieren der organischen Phase mit 100 mg 2,6-Di-tert.butylkresol konnte diese zu 181.00 g (79 % d.Th.) eines hellen hochviskosen Öles von Trimellitsäure-N-tert.butyl-N-methacryloyloxyethylamid (IX) eingeengt werden. Das erhaltene Öl erstarrte beim Abkühlen und ließ sich weiter reinigen, indem man es aus einer Dichlormethanlösung mit wäßriger Natriumcarbonatlösung extrahierte, die wäßrige Phase mit verdünnter Salzsäure ansäuerte und das Produkt (IX) in eine Dichlormethanphase zurück extrahierte. Schmelzpunkt: 74°C
- IR (KBr):: ν= 3500-2500, 2950, 1720, 1630, 1600, 1400, 1365, 1290, 1192, 1158 cm⁻¹.
- ¹H-NMR: (CDCl₃, 200 MHz): δ = 1,58 (s, 9 H, C(CH₃)₃), 1,90 (bs, 3 H, = CCH₃), 3,65 (t, J = 6,0 Hz, 2 H, NCH₂), 4,13 (t, J = 6,0 Hz, 2 H, OCH₂), 5,61, 6,05 (2 m, je 1 H, vinyl.-H), 7,52 (m, 3 H, arom.-H, COOH), 7,82 (m, 1 H, arom. H) ppm.

MS (70 eV-silyliert): m/z = 506 (M-CH₃), 435 (M-C₃H₅COOH),
379 (M-C₃H₅COOH-C₄H₈), 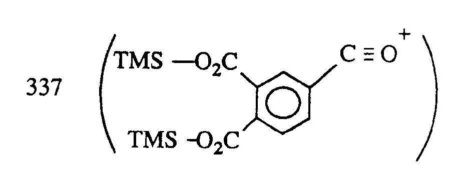
147 (Me₃Si-O-SiMe₂), 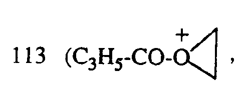
73 (TMS⁺), 69 (C₃H₅-C≡O⁺), 57 (C₄H₉⁺).

### Beispiel 3:

Pyromellitsäure-1,4-di(N-tert.butyl-N-methacryloyloxyethylamid) (X)
Pyromellitsäure-1,3-di(N-tert.butyl-N-methacryloyloxyethylamid) (XI)

Unter starkem Rühren wurde bei Raumtemperatur zu einer Suspension aus 218.12 g (1.000 mol) Pyromellitsäuredianhydrid, 750 ml trockenem Dichlormethan und 505.95 g (5.000 mol) trockenem Triethylamin eine Lösung von 370.54 g (2.000 mol) N-tert.Butyl-2-aminoethylmethacrylat in 370 ml trockenem Dichlormethan getropft. Hierbei entstand unter leichter Erwärmung eine braune Lösung, die eine Stunde lang bei 41°C gerührt, in 1 l Wasser eingegossen und mit halbkonzentrierter Schwefelsäure angesäuert wurde.

Nach Abtrennung des ausgefallenen Feststoffes wurde die Dichlormethanphase wäßrig extrahiert, getrocknet und zu 314.70 g (53 % d. Th.) eines beigefarbenen Feststoffes eines Gemisches der para- und meta-Isomeren (X) und (XI) des Pyromellitsäuredi(N-tert.butyl-N-methacryloyloxyethylamids) eingeengt. Durch Aufnehmen des erhaltenen Produktes in wäßriger Natriumcarbonatlösung, Extraktion mit Methylenchlorid und erneutes Ausfällen mit halbkonzentrierter Schwefelsäure wurde ein gereinigter weißer Feststoff erhalten.

Schmelzpunkt: 121°C
- IR (KBr) :: ν= 3400-2500, 1715, 1618, 1411, 1405, 1360, 1330, 1300, 1213, 1172, 1131, 955, 780 cm⁻¹.
- ¹H-NMR: (CDCl₃, 200 MHz): δ = 1,57 (s, 18 H, C(CH₃)₃), 1,86 (bs, 6 H, = CCH₃), 3,45 (m, 4 H, NCH₂), 4,12 (m, 4 H, OCH₂), 5,53, 5,98 (2 m, je 2 H, vinyl.-H), 7,89 (bs, 2 H, COOH), 8,41 (s, 2H, arom.-H) ppm.

- FAB-MS (Glycerin-DMF): m/z =: 589 ([M+H]⁺)
587 ([M-H]⁻).

### Beispiel 4:

Pyromellitsäure-(N-tert.butyl-N-methacryloyloxyethylamid) (XII)

Eine Mischung aus 218.12 g (1.000 mol) Pyromellitsäuredianhydrid, 171.50 g (1.200 mol) Triethylamin und 2200 ml Xylol wurde bei Raumtemperatur unter Rühren mit einer Lösung von 185.27 g (1.000 mol) N-tert.Butyl-2-aminoethylmethacrylat in 250 ml Xylol versetzt und eine Stunde lang auf 50°C erwärmt. Der Ansatz wurde filtriert und das Filtrat in Eiswasser eingegossen und zur Hydrolyse mit halbkonzentrierter Schwefelsäure angesäuert und 30 Minuten verrührt. Der ausgefallene Niederschlag wurde abgesaugt und aus Essigsäureethylester zu 232.05 g (etwa 55 % d. Th.) eines weißen Feststoffes umkristallisiert, der neben dem Pyromellitsäure-(N-tert.butyl-N-methacryloyloxyethylamid) (XII) eine kleinere Menge der doppelten Umsetzungsprodukte (X) und (XI) des Beispieles 3 enthielt.

Schmelzpunkt: 215°C
- IR (KBr):: ν= 3500-2500, 1710, 1605, 1400, 1345, 1316, 1282, 1194, 1153, 932, 752 cm⁻¹.
- ¹H-NMR: (d₆-DMSO, 200 MHz): δ = 1.49 (s, 9H, C(CH₃)₃), 1.78 (bs, = C-CH₃), 3.44 (m, 2 H, NCH₂), 4.06 (m, 2 H, OCH₂), 5.61, 5.89 (2 m, je 1 H, vinyl.-H), 7.62, 8.29(2 s, je 1 H, arom. H), 8.40 (bs, 3 H, COOH) ppm.

- FAB-MS (Glycerin-DMF): m/z =: 422 ([M+H]⁺),
420 ([M-H]⁻).

### Beispiel 5:

Benzol-1,3-dicarbonsäure-4-(carbonsäure-N-tert.butyl-N-methacryloyloxyethylamid) (XIII)
Benzol-1,4-dicarbonsäure-5-(carbonsäure-N-tert.butyl-N-methacryloyloxyethylamid) (XIV)

Zu einer Mischung aus 192.13 g (1.000 mol) Benzoltricarbonsäureanhydrid, 500 ml trockenem Dichlormethan und 303.57 g (3.000 mol) trockenem Triethylamin wurde bei Raumtemperatur unter Rühren eine Lösung von 185.27 g (1.000 mol) N-tert.-Butyl-2-aminoethylmethacrylat in 250 ml Dichlormethan getropft. Nach vierstündigem Rühren bei 41°C wurde der Ansatz in 3 l Wasser gegossen, mit halbkonzentrierter Schwefelsäure angesäuert und die organische Phase abgetrennt. Die wäßrige Phase wurde mit Dichlormethan extrahiert und die vereinigten organischen Phasen nach Trocknung und Stabilisierung mit 350 mg 2,6-Di-tert.-butylkresol zu 284.60 g (78 % d. Th.) eines beigefarbenen Feststoffes eines Gemisches der beiden Isomeren (XIII) und (XIV) des Benzoltricarbonsäuremono(N-tert.butyl-N-methacryloyloxyethylamides) eingeengt.

Schmelzpunkt: 71°C
- IR (KBr):: ν= 3400-2400, 1700, 1610, 1370, 1283, 1161, 1040, 1010, 932, 752 cm-1.
- ¹H-NMR: (CDCl₃, 200 MHz): für XIII: δ = 1.58 (bs, 9 H, C(CH₃)₃, 1.82 (bs, 3 H, =CCH₃), 3.32 (m, 2 H, NCH₂), 4.51 (m, 2 H, OCH₂), 5.49, 5.95(2 m, je 1 H, vinyl.-H), 7.1 (m, 2 H, COOH), 8.15, 8.75 (m, 3 H, arom. H) ppm.

für XIV : δ = 1.43 (s, 9 H, C(CH₃)₃), 1.8 (bs, 3 H, =CCH₃), 3.48 (m, 2 H, NCH₂), 4.1 (m, 2 H, OCH₂), 5.44, 6.03 (2 m, je 1 H, vinyl.-H), 7.1 (m, 2 H, COOH), 8.0 - 8.6 (m, 3 H, arom. H) ppm.

### Beispiele 6 bis 10:

(Herstellung der Zubereitungen zur Verwendung als Adhäsiv)

Die erfindungsgemäßen Adhäsive werden durch intensives Vermischen der in folgenden Beispielen aufgeführten Bestandteile erzeugt.

### Beispiel 6

- 37.5 g: Wasser
- 50.0 g: Tetrahydrofuran
- 12.5 g: Trimellitsäure-N-tert.butyl-N-methacryloyloxyethylamid (IX) gemäß Beispiel 2
- 0.02 g: Campherchinon

### Beispiel 7

- 27.3 g: Wasser
- 63.6 g: Tetrahydrofuran
- 9.1 g: Trimellitsäure-N-tert.butyl-N-methacryloyloxyethylamidanhydrid (VIII) gemäß Beispiel 1
- 0.02 g: Campherchinon

### Beispiel 8

- 40.0 g: Wasser
- 48.0 g: Tetrahydrofuran
- 8.4 g: Trimellitsäure-N-tert.butyl-N-methacryloyloyxethylamid (IX) gemäß Beispiel 2
- 3.6 g: Trimellitsäure-N-tert.butyl-N-methacryloyloxyethylamidandhydrid (VIII) gemäß Beispiel 1
- 0.02 g: Campherchinon

### Beispiel 9

- 93.7 g: Ethanol
- 6.3 g: Pyromellitsäuredi(N-tert.butyl-N-methacryloyloxyethylamid) (p-und m-Isomere (X) und (XI) gemäß Beispiel 3)
- 0.01 g: Campherchinon

### Beispiel 10

- 43.5 g: Wasser
- 43.5 g: Tetrahydrofuran
- 9.5 g: Pyromellitsäure-(N-tert.butyl-N-methacryloyloxyethylamid) (XII) gemäß Beispiel 4
- 3.5 g: Pyromellitsäuredi(N-tert.butyl-N-methacryloyloxyethylamid) (p- und m-Isomere (X) und (XI) gemäß Beispiel 3)
- 0.02 g: Campherchinon

### Beispiel 11 (Anwendungstest, Bindungsfestigkeit)

Die Wirksamkeit und Eignung der Adhäsive (Beispiele 6 - 10) wird überprüft durch Bestimmung der Scherbindungsfestigkeit auf Dentin. Es werden menschliche Zähne benutzt, die für max. drei Monate nach der Extraktion in 1% Chloraminlösung aufbewahrt waren. Vor der Verwendung im Test werden die Zähne nach sorgfältiger Reinigung unter fließendem Wasser für mindestens drei und höchstens zehn Tage in physiologischer Kochsalzlösung gelagert. Am Tage vor der Verwendung im Bindungstest werden die Zähne einzeln auf einer Approximalseite liegend mit Epoxidharz (®LEKUTHERM X 20, Härter T 3) in zylindrische Gummiformen von 25 mm Durchmesser und 12 mm Höhe eingebettet. Die Zähne werden durch Naßschleifen auf SiC-Papieren der Körnungen 240, 320, 400 und schließlich 600 soweit beschliffen, daß eine ausreichend große schmelznahe Dentinfläche zur Anbindung eines Kunststoffzylinders mit 3,5 mm Durchmesser freiliegend. Nach Abspülen mit entionisiertem Wasser und Trocknung im Luftstrom wird 30 Sekunden lang die Konditionierlösung ®GLUMA 1 Cleanser mit einem Wattepellet unter reibender Bewegung aufgetragen, mit Wasser abgespült und getrocknet, bevor das Adhäsiv mit einem Pinsel aufgetragen, 30 Sekunden auf der Oberfläche belassen und dann im Druckluftstrom vorsichtig getrocknet wird. Anschließend wird ein Tropfen ®GLUMA 4 Sealer aufgetragen und mit Druckluft zu einer dünnen Schicht verblasen. Die so vorbehandelte Probe wird in einer Einspannvorrichtung unter einer teilbaren Teflonform mit einer zylindrischen 3,5 mm weiten und 1 mm hohen Aufnahme festgeklemmt. Danach wird das Kunststoff-Füllungsmaterial ®PEKAFILL (U) mit einer Spritze in die zylindrische Form eingefüllt, mit einem O₂-undurchlässigen Strip abgedeckt und mit der Polymerisationsleuchte ®TRANSLUX CL (Kulzer) unter der aufliegenden Lichtaustrittsöffnung 60 Sekunden lang aktiviert. Unmittelbar im Anschluß daran wird die Probe aus der Halterung entfernt. Die Teflonform wird abgenommen und die Probe wird für 15 Minuten in 23°C warmem Wasser gelagert bis zur Einleitung der Scherbelastung, die mit Hilfe eines Druckstempels parallel zu und dicht an der Oberfläche des eingebetteten Zahnes unter einer Vorschubgeschwindigkeit von 1 mm/Minute bis zur Abtrennung erfolgt. Die Scherbindungsfestigkeit an Dentin ist der Quotient aus Druckkraft und Kontaktareal am Zahn und wird jeweils an 5 Proben bestimmt und als deren Mittelwert und Standardabweichung angegeben.

Zur Beurteilung der Frakturursache wird die Dentinseite der getrennten Proben im Auflichtmikroskop inspiziert.

### Beispiel 12 (Anwendungstest, Zahnkavität)

Zur Simulation der klinischen Anwendung von Adhäsiven und Kunststoff-Füllungsmaterialien werden in extrahierten Zähnen mit einer Vorgeschichte wie in Beispiel 11 Kavitäten präpariert und gefüllt. Als Maß für die Wirksamkeit wird die Adaption des Füllungsmaterials am Kavitätenrand bestimmt.

Die extrahierten Zähne werden auf einer unbeschädigten Aproximalseite auf SiC-Papier der Körnungen 240, 320, 400 und 600 naß beschliffen bis eine ausreichend große Dentinfläche zur Aufnahme einer ca. 3 mm weiten zylindrischen Kavität freiliegt. Die Kavität wird mit üblichen, zahnärztlichen Präparationsdiamanten mittlerer Körnung unter reichlich Wasserkühlung bis zu einer Tiefe von ca. 1,5 mm präpariert, dann mit Wasser ausgespült und getrocknet. Mit einem getränkten Wattepellet wird wie im vorhergegangenen Beispiel 30 Sekunden lang die Kavität gereinigt, dann ausgewaschen und getrocknet bevor das Adhäsiv aufgepinselt, für 30 Sekunden belassen und schließlich getrocknet wird. Anschließend wird der ®GLUMA 4 Sealer aufgebracht. Mit Druckluft wird vorsichtig der Überschuß entfernt bevor das Kunststoff-Füllungsmaterial ®PEKAFILL (U) mit einer Spritze in die Kavität eingefüllt wird. Der Überschuß wird mit einem O₂-undurchlässigem Strip vor Aktivierung (60 Sekunden) mit dem Lichtpolymerisationsgerät ®TRANSLUX CL (Kulzer) abgedeckt. Unmittelbar nach der Polymerisation wird der gefüllte Zahn für 15 Minuten in 23°C warmem Wasser aufbewahrt. Im Anschluß daran wird der Überschuß durch Schleifen auf feuchtem SiC-Papier der Körnungen 400 und 600 entfernt. Dabei werden ca. 0,1 mm von der Kavitätenhöhe abgetragen. Der mit Wasser abgespülte Zahn wird im Luftstrom getrocknet und sofort im Auflichmikroskop bei 500-facher Vergrößerung inspiziert. Die maximale Breite eines etwa vorhandenen Randspaltes wird mit Hilfe eines Schraubenokularmikrometers vermessen. Die mittlere, maximale Spaltbreite von je 5 Füllungen wird als Meßwert angegeben. Die mikroskopische Untersuchung des einzelnen Zahnes war in allen Fällen in weniger als 10 Minuten abgeschlossen. Damit wurde sichergestellt, daß die gemessenen Randspalten nicht durch Dehydrierung des Dentins entstanden oder in ihrer Breite beeinflußt waren.

### Ergebnisse der anwendungstechnischen Untersuchungen

Mit dem in Beispielen 11 und 12 beschriebenen Anwendungstest ließ sich die gute Wirksamkeit der Zubereitung gemäß der Beispiele 6 bis 10 nachweisen.

Bei der lichtmikroskopischen Beurteilung der Frakturursache wurden vielfach Kohäsivfrakturen im Dentin oder im Kunststoff beobachtet, d.h. die mit den erfindungsgemäßen Adhäsivkomponenten erzeugten Verklebungen waren fester als die verklebten Fügeteile selbst. Dies zeigt die gute Leistungsfähigkeit der erfindungsgemäßen Adhäsivkomponenten.

Mit der Zubereitung gemäß Beispiel 6 wurden nach der beschriebenen Methode sogar ausschließlich randspaltfreie Füllungen erhalten. Die Scherbindungsfestigkeit wurde formal zu 12.1 ± 3.5 N/mm² bestimmt, wobei wie oben erwähnt die Fraktur im Dentin oder Kunststoff erfolgte, also kein adhäsives Versagen erreichbar war.

## Patentansprüche (Patentansprüche für folgende(n) Vertragsstaat(en): AT, BE, CH, DE, DK, FR, GB, GR, IT, LI, LU, NL, SE)

1. N-Alkyl-N-(meth)acryloyloxyalkylcarboxamide der Formel (I) 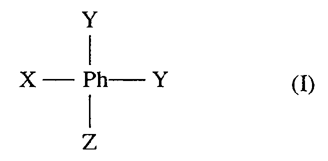 in der
X für N-Alkyl-N-(meth)acryloyloxyalkylcarboxamid der Formel 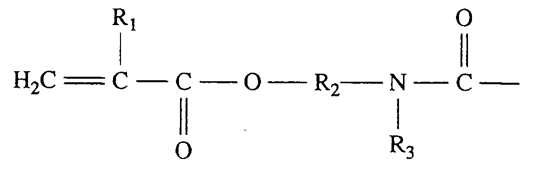 steht, worin
R₁ Wasserstoff oder Methyl bedeutet,
R₂ ein zweiwertiger aliphatischer Rest (C₂-C₆) bedeutet und
R₃ ein einwertiger aliphatischer Rest (C₁-C₄) bedeutet,
Y für COOH steht, wobei nachbarständige Gruppen Y auch zu einer Anhydridgruppe ( -CO-O-CO-) verknüpft sein können,
Z für H, X, oder, sofern Y COOH bedeutet, für Y steht und
Ph für einen drei- oder vierfach substituierten Benzolring (1,2,3-/1,2,4- oder 1,2,4,5-Substitution) oder einen drei- oder vierfach substituierten Naphthalinring (1,2,6-/1,4,5-/2,3,6-/1,4,5,8- oder 2,3,6,7-Substitution)
steht.

2. Verfahren zur Herstellung der N-Alkyl-N-(meth)acryloyloxyalkylcarboxamide gemäß Anspruch 1 dadurch gekennzeichnet, daß man
Monoanhydride der Formel (V) 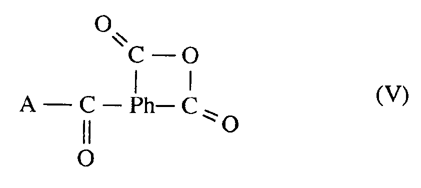 oder Dianhydride der Formel (VI) 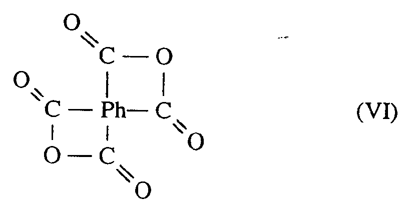 worin
A Hydroxy (OH) oder Chlor (Cl) bedeutet und
Ph für einen drei- oder vierfach substituierten Benzolring (1,2,3-/1,2,4- oder 1,2,4,5-Substitution) oder einen drei- oder vierfach substituierten Naphthalinring (1,2,6-/1,4,5-/2,3,6-/1,4,5,8- oder 2,3,6,7-Substitution)
steht
in einem organischen Lösungsmittel zwischen -30 und 110°C gegebenenfalls in Gegenwart einer Base
mit einem Amin der Formel (VII) 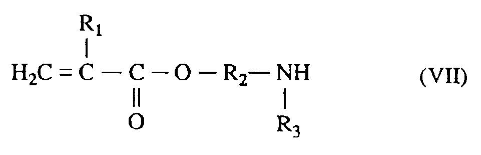 in der
R₁ Wasserstoff oder Methyl,
R₂ einen zweiwertigen aliphatischen Rest (C₂-C₆) und
R₃ einen einwertigen aliphatischen Rest (C₁-C₄) bedeutet,
umsetzt und das erhaltene Produkt gegebenenfalls mit Wasser zwischen 5 und 100°C hydrolysiert.

3. Zubereitungen enthaltend Carboxamide gemäß Anspruch 1, ein Lösungsmittel und gegebenenfalls einen Initiator.

4. Zubereitungen gemäß Anspruch 3, dadurch gekennzeichnet, daß als Initiator ein Radikalbildner aus der Reihe der Mono- oder Dicarbonylverbindungen und gegebenenfalls ein Coaktivator zugesetzt wird.

5. Zubereitungen nach den Ansprüchen 3 und 4, dadurch gekennzeichnet, daß als weitere Komponente ein (Meth)acrylsäureester, der Vernetzungen ausbilden kann, zugesetzt wird.

6. Verwendung von Zubereitungen enthaltend N-Alkyl-N-(meth)arcyloyloxyalkylcarboxamide gemäß Anspruch 1 als Adhäsivkomponente zur Behandlung von Zahnhartsubstanz.

7. Verwendung von Zubereitungen nach Anspruch 6 im Anschluß an eine Konditionierung der Zahnhartsubstanz mit einer Flüssigkeit eines pH-Wertes von 0,1 bis 3,5.

8. Verwendung nach Ansprüchen 6 und 7 als Klebemittel zur Befestigung von Zahnreparaturmaterialien am Zahn.

9. Verwendung nach Anspruch 6 als Klebemittel in Knochenzement.

## Patentansprüche (Patentansprüche für folgende(n) Vertragsstaat(en): ES)

1. Verfahren zur Herstellung von N-Alkyl-N-(meth)acryloyloxyalkylcarboxamiden der Formel (I) 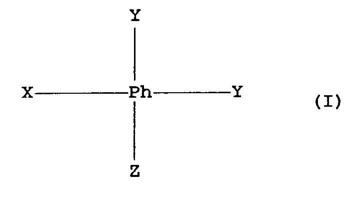 in der
X für N-Alkyl-N-(meth)acryloyloxyalkylcarboxamid der Formel 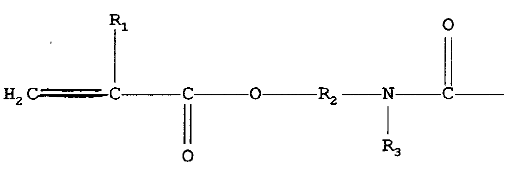 steht, worin
R₁ Wasserstoff oder Methyl bedeutet,
R₂ ein zweiwertiger aliphatischer Rest (C₂-C₆) bedeutet und
R₃ ein einwertiger aliphatischer Rest (C₁-C₄) bedeutet,
Y für COOH steht, wobei nachbarständige Gruppen Y auch zu einer Anhydridgruppe (-CO-O-CO-) verknüpft sein können,
Z für H, X oder, sofern Y COOH bedeutet, für Y steht und
Ph für einen drei- oder vierfach substituierten Benzolring (1,2,3-/1,2,4- oder 1,2,4,5-Substitution) oder einen drei- oder vierfach substituierten Naphthalinring (1,2,6-/1,4,5-/2,3,6-/1,4,5,8- oder 2,3,6,7-Substitution)
steht, dadurch gekennzeichnet, daß man Monoanhydride der Formel (V) 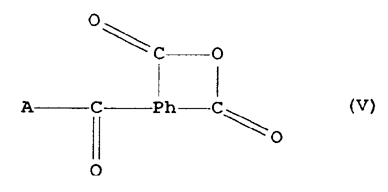 oder Dianhydride der Formel (VI) 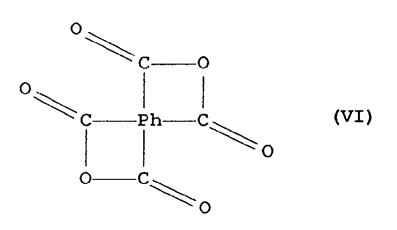 worin
A Hydroxy (OH) oder Chlor (Cl) bedeutet und
Ph für einen drei- oder vierfach substituierten Benzolring (1,2,3-/1,2,4- oder 1,2,4,5-Substitution) oder einen drei- oder vierfach substituierten Naphthalring (1,2,6-/1,4,5-/2,3,6-/1,4,5,8- oder 2,3,6,7-Substitution)
steht
in einem organischen Lösungsmittel zwischen -30 und 110°C gegebenenfalls in Gegenwart einer Base mit einem Amin der Formel (VII) 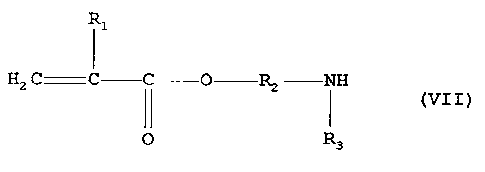 in der
R₁ Wasserstoff oder Methyl,
R₂ einen zweiwertigen aliphatischen Rest (C₂-C₆) und
R₃ einen einwertigen aliphatischen Rest (C₁-C₄) bedeutet,
umsetzt und das erhaltene Produkt gegebenenfalls mit Wasser zwischen 5 und 100°C hydrolysiert.

## Claims (Claims for the following Contracting State(s): AT, BE, CH, DE, DK, FR, GB, GR, IT, LI, LU, NL, SE)

1. N-Alkyl-N-(meth)acryloyloxyalkylcarboxamides of the formula (I) 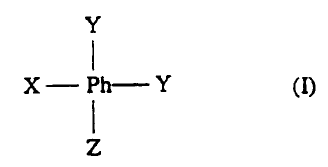 in which
X represents N-alkyl-N-(meth)acryloyloxyalkylcarboxamide of the formula 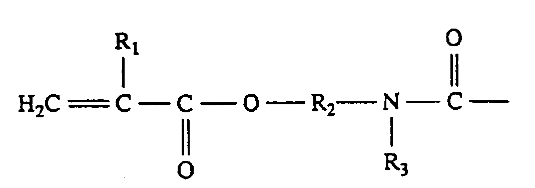 wherein
R₁ denotes hydrogen or methyl,
R₂ denotes a divalent aliphatic radical (C₂-C₆) and
R₃ denotes a monovalent aliphatic radical (C₁-C₄),
Y represents COOH, and wherein adjacent groups Y can also be linked to form an anhydride group (-CO-O-CO-),
Z represents H, X or, if Y denotes COOH, represents Y and
Ph represents a tri- or tetrasubstituted benzene ring (1,2,3/1,2,4 or 1,2,4,5 substitution) or a tri- or tetrasubstituted naphthalene ring (1,2,6/1,4,5/ 2,3,6/1,4,5,8 or 2,3,6,7 substitution).

2. Process for the preparation of the N-alkyl-N-(meth)acryloyloxyalkylcarboxamides according to Claim 1, characterized in that
monoanhydrides of the formula (V) 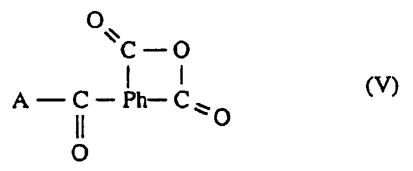 or dianhydrides of the formula (VI) 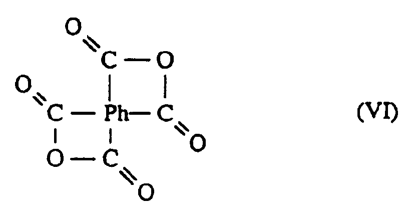 wherein
A denotes hydroxyl (OH) or chlorine (Cl) and
Ph represents a tri- or tetrasubstituted benzene ring (1,2,3/1,2,4 or 1,2,4,5 substitution) or a tri- or tetrasubstituted naphthalene ring (1,2,6/1,4,5/ 2,3,6/1,4,5,8 or 2,3,6,7 substitution)
are reacted with an amine of the formula (VII) 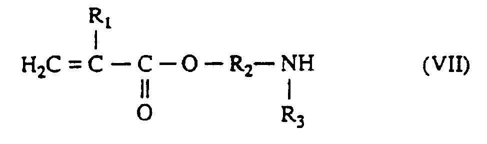 in which
R₁ denotes hydrogen or methyl,
R₂ denotes a divalent aliphatic radical (C₂-C₆) and
R₃ denotes a monovalent aliphatic radical (C₁-C₄),
in an organic solvent at between -30 and 110°C, if appropriate in the presence of a base, and, if appropriate, the resulting product is hydrolysed with water at between 5 and 100°C.

3. Formulations comprising carboxamides according to Claim 1, a solvent and, if appropriate, an initiator.

4. Formulations according to Claim 3, characterized in that an agent which forms free radicals from the series comprising mono- and dicarbonyl compounds and, if appropriate, a coactivator are added as the initiator.

5. Formulations according to Claims 3 and 4, characterized in that a (meth)acrylic acid ester which can form crosslinkings is added as a further component.

6. Use of formulations comprising N-alkyl-N-(meth)-acryloyloxyalkylcarboxamides according to Claim 1 as adhesive component for the treatment of the hard substance of teeth.

7. Use of formulations according to Claim 6 after conditioning of the hard substance of teeth with a liquid having a pH of 0.1 to 3.5.

8. Use according to Claims 6 and 7 as an adhesive for fixing dental repair materials to the tooth.

9. Use according to Claim 6 as an adhesive in bone cement.

## Claims (Claims for the following Contracting State(s): ES)

1. Process for the preparation of N-alkyl-N-(meth)-acryloyloxyalkylcarboxamides of the formula (I) 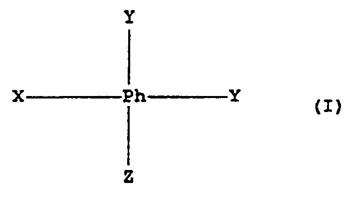 in which
X represents N-alkyl-N-(meth)acryloyloxyalkylcarboxamide of the formula 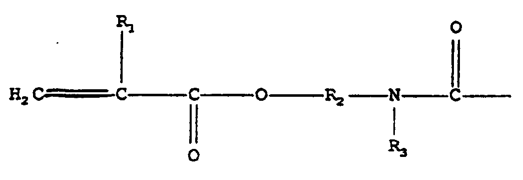 wherein
R₁ denotes hydrogen or methyl,
R₂ denotes a divalent aliphatic radical (C₂-C₆) and
R₃ denotes a monovalent aliphatic radical (C₁-C₄),
Y represents COOH, and wherein adjacent groups Y can also be linked to form an anhydride group (-CO-O-CO-),
Z represents H, X or, if Y denotes COOH, represents Y and
Ph represents a tri- or tetrasubstituted benzene ring (1,2,3/1,2,4 or 1,2,4,5 substitution) or a tri- or tetrasubstituted naphthalene ring (1,2,6/1,4,5/ 2,3,6/1,4,5,8 or 2,3,6,7 substitution),
characterized in that monoanhydrides of the formula (V) 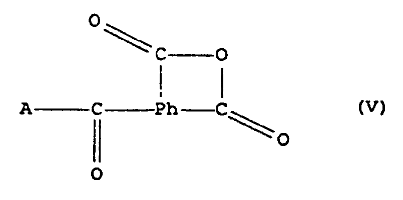 or dianhydrides of the formula (VI) 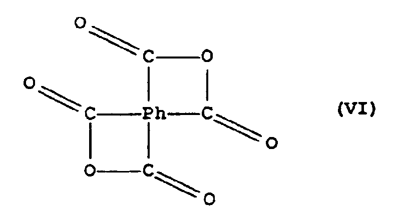 wherein
A denotes hydroxyl (OH) or chlorine (Cl) and
Ph represents a tri- or tetrasubstituted benzene ring (1,2,3/1,2,4 or 1,2,4,5 substitution) or a tri- or tetrasubstituted naphthalene ring (1,2,6/1,4,5/ 2,3,6/1,4,5,8 or 2,3,6,7 substitution)
are reacted with an amine of the formula (VII) 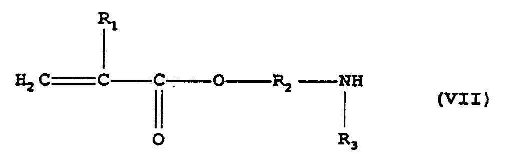 in which
R₁ denotes hydrogen or methyl,
R₂ denotes a divalent aliphatic radical (C₂-C₆) and
R₃ denotes a monovalent aliphatic radical (C₁-C₄),
in an organic solvent at between -30 and 110°C, if appropriate in the presence of a base, and, if appropriate, the resulting product is hydrolysed with water at between 5 and 100°C.

## Revendications (Revendications pour l'(les) Etat(s) contractant(s) suivant(s): AT, BE, CH, DE, DK, FR, GB, GR, IT, LI, LU, NL, SE)

1. N-alkyl-N-(méth)acryloyloxyalkylcarboxamides de formule (I) 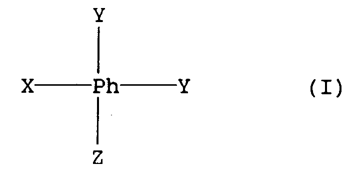 dans laquelle
X représente un N-alkyl-N-(méth)acryloyloxyalkylcarboxamide de formule 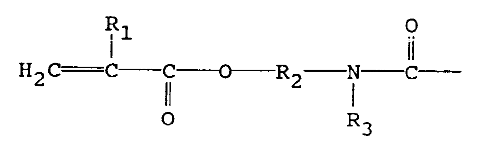 dans laquelle
R₁ représente un atome d'hydrogène ou un groupe méthyle,
R₂ représente un radical aliphatique bivalent en C₂-C₆, et
R₃ représente un radical aliphatique monovalent en C₁-C₄,
Y représente COOH, des groupes Y adjacents pouvant également être reliés en un groupe anhydride (-CO-O-CO-),
Z représente H, X ou, pour autant que Y représente COOH, représente Y, et
Ph représente un cycle benzénique trois ou quatre fois substitué (substitution aux positions 1,2,3/1,2,4 ou 1,2,4,5) ou encore un noyau naphtalène substitué trois ou quatre fois (substitution aux positions 1,2,6/1,4,5/2,3,6/1,4,5,8 ou 2,3,6,7).

2. Procédé pour la préparation des N-alkyl-N-(méth)acryloyloxyalkylcarboxamides selon la revendication 1, caractérisé en ce qu'on fait réagir des monoanhydrides de formule (V) 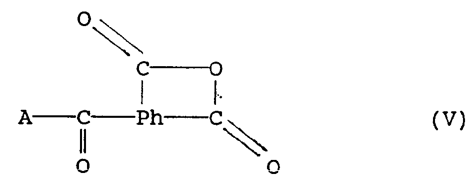 ou des dianhydrides de formule (VI) 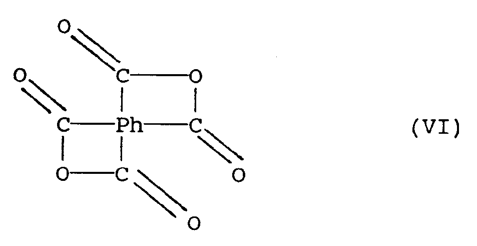 dans lesquelles
A représente un groupe hydroxyle (OH) ou un atome de chlore (Cl) et
Ph représente un cycle benzénique trois ou quatre fois substitué (substitution aux positions 1,2,3/1,2,4 ou 1,2,4,5) ou encore un noyau naphtalène substitué trois ou quatre fois (substitution aux positions 1,2,6/1,4,5/2,3,6/1,4,5,8 ou 2,3,6,7),
dans un solvant organique, entre -30 et 110°C, éventuellement en présence d'une base,
avec une amine de formule (VII) 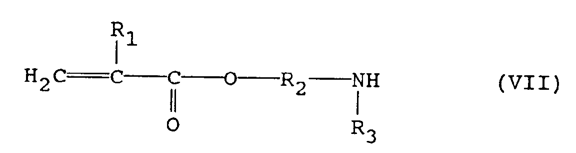 dans laquelle
R₁ représente un atome d'hydrogène ou un groupe méthyle,
R₂ représente un radical aliphatique bivalent en C₂- C₆, et
R₃ représente un radical aliphatique monovalent en C₁-C₄,
et, éventuellement, on hydrolyse le produit obtenu avec de l'eau entre 5 et 100°C.

3. Préparations contenant des carboxamides selon la revendication 1, un solvant et éventuellement un initiateur.

4. Préparations selon la revendication 3, caractérisées en ce que, comme initiateur, on ajoute un formateur de radicaux de la série des composés de mono- ou dicarbonyle et éventuellement un coactivateur.

5. Préparations selon les revendications 3 et 4, caractérisées en ce que, comme composant supplémentaire, on ajoute un ester (méth)acrylique qui peut former des réticulations.

6. Mise au point de préparations contenant des N-alkyl-N-(méth)acryloyloxyalkylcarboxamides selon la revendication 1, comme composant d'adhésif pour le traitement de la substance dure des dents.

7. Utilisation des préparations selon la revendication 6, suite à un conditionnement de la substance dure des dents avec un liquide ayant un pH de 0,1 à 3,5.

8. Utilisation selon les revendications 6 et 7, comme adhésif pour fixer des matières de réparation dentaire sur la dent.

9. Utilisation selon la revendication 6, comme adhésif dans du ciment d'os.

## Revendications (Revendications pour l'(les) Etat(s) contractant(s) suivant(s): ES)

1. Procédé pour la préparation de N-alkyl-N-(méth)acryloyloxyalkylcarboxamides de formule (I) 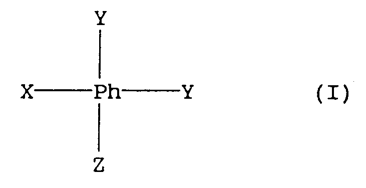 dans laquelle
X représente un N-alkyl-N-(méth)acryloyloxyalkyl- carboxamide de formule 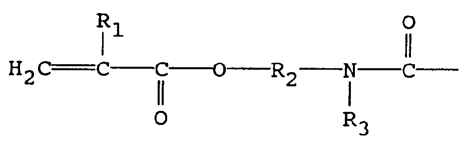 dans laquelle
R₁ représente un atome d'hydrogène ou un groupe méthyle,
R₂ représente un radical aliphatique bivalent en C₂-C₆, et
R₃ représente un radical aliphatique monovalent en C₁-C₄,
Y représente COOH, des groupes Y adjacents pouvant également être reliés en un groupe anhydride (-CO-O-CO-),
Z représente H, X ou, pour autant que Y représente COOH, représente Y, et
Ph représente un cycle benzénique trois ou quatre fois substitué (substitution aux positions 1,2,3/1,2,4 ou 1,2,4,5) ou encore un noyau naphtalène substitué trois ou quatre fois (substitution aux positions 1,2,6/1,4,5/2,3,6/1,4,5,8 ou 2,3,6,7),
caractérisé en ce qu'on fait réagir des monoanhydrides de formule (V) 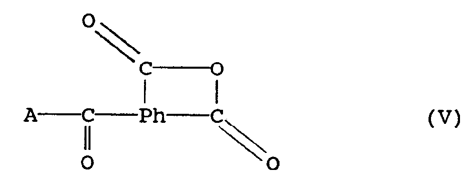 ou des dianhydrides de formule (VI) 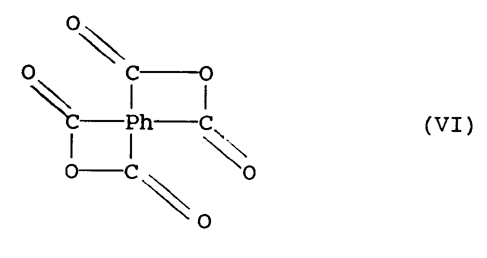 dans lesquelles
A représente un groupe hydroxyle (OH) ou un atome de chlore (Cl) et
Ph représente un cycle benzénique trois ou quatre fois substitué (substitution aux positions 1,2,3/1,2,4 ou 1,2,4,5) ou encore un noyau naphtalène substitué trois ou quatre fois (substitution aux positions 1,2,6/1,4,5/2,3,6/1,4,5,8 ou 2,3,6,7),
dans un solvant organique, entre -30 et 110°C, éventuellement en présence d'une base,
avec une amine de formule (VII) 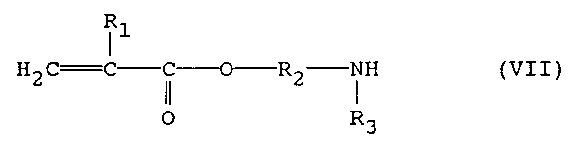 dans laquelle
R₁ représente un atome d'hydrogène ou un groupe méthyle,
R₂ représente un radical aliphatique bivalent en C₂-C₆, et
R₃ représente un radical aliphatique monovalent en C₁-C₄,
et, éventuellement, on hydrolyse le produit obtenu avec de l'eau entre 5 et 100°C.
